# EUROPÄISCHE PATENTSCHRIFT

(11) **EP 1 804 862 B1**
(45) Veröffentlichungstag und Bekanntmachung des Hinweises auf die Patenterteilung: **18.01.2012**
(21) Anmeldenummer: 05799629.0
(22) Anmeldetag: 25.10.2005
(51) Int. Cl.: A61M 5/28, A61M 5/50, A61M 5/24, A61M 5/31

(54) **VERFAHREN UND VORRICHTUNGEN ZUM LYOPHILISIEREN, REKONSTITUIEREN UND VERABREICHEN EINES REKONSTITUIERTEN WIRKSTOFFES**
METHOD AND DEVICES FOR LYOPHILIZING, RECONSTITUTING, AND ADMINISTERING A RECONSTITUTED AGENT
PROCEDES ET DISPOSITIFS DE LYOPHILISATION, DE RECONSTITUTION ET D'ADMINISTRATION D'UN PRINCIPE ACTIF RECONSTITUANT

(30) Priorität: 25.10.2004 AT 18042004
(43) Veröffentlichungstag der Anmeldung: 11.07.2007
(73) Patentinhaber: Pharma Consult Ges.m.b.H. & Co Nfg KG, A-1210 Wien (AT)
(72) Erfinder: PICKHARD, Ewald, A-2203 Grossebersdorf (AT)
(74) Vertreter: Burger, Hannes
(86) Internationale Anmeldenummer: PCT/AT2005/000423
(87) Internationale Veröffentlichungsnummer: WO 2006/045132

(56) Entgegenhaltungen:
- EP-A- 1 393 763
- EP-A- 1 459 775
- WO-A-01/00261
- WO-A-97/47343
- WO-A-02/072171
- US-A- 4 254 768
- US-A- 4 941 876
- US-A- 5 429 603
- US-A- 5 637 087
- US-A- 5 788 670
- US-A1- 2004 116 874

## Beschreibung

Die Erfindung bezieht sich auf eine Vorrichtung sowie auf ein Verfahren zur Lyophilisation, Rekonstitution und Verabreichung von Wirkstoffen.

Es sind bereits Verfahren zum Lyophilisieren, Rekonstituieren und Verabreichen von lyophilisierten Wirkstoffen bekannt.

Aus der WO 01/00261 A1 ist eine Vorrichtung zum Lyophilisieren, rekonstituieren und verabreichen eines rekonstitutierten Wirkstoffes bekannt geworden. Dabei weist die Vorrichtung ein längliches Gehäuse mit einem abgabeseitigen vorderen Gehäuseende und einem diesen gegenüberliegenden hinteren Gehäuseende auf. Innerhalb des Gehäuses ist im Bereich des vorderen Gehäuseendes eine erste Kammer mit einem darin enthaltenen Lyophilisat ausgebildet. Dabei ist die erste Kammer an ihrem den vorderen Gehäuseende zugewendeten Ende mit einem abnehmbaren Verschluss und in Richtung des hinteren Gehäuseendes mit einem Kolben dichtend verschlossen. Weiters ist im Gehäuse im Bereich des hinteren Gehäuseendes eine zweite Kammer ausgebildet, wobei die zweite Kammer über eine Öffnung mit der umgebenden Atmosphäre verbunden ist. Die Membrane ist derart ausgebildet, dass diese für Gas, jedoch nicht für Bakterien, durchlässig ist. In der zweiten Kammer ist eine ein Lösungsmittel enthaltende Karpule angeordnet. Um eine Verbindung zwischen dem Inneren der Karpule und der ersten Kammer herstellen zu können, sind zwischen der ersten Kammer und der Karpule eigene Verbindungsmittel vorgesehen.

So ist es aus der US 5,637,087 A bekannt, einen versiegelten, ersten Wirkstoffbehälter zu verwenden, indem ein lyophilisierter Wirkstoff enthalten ist. Dieser Wirkstoffbehälter kann gegebenenfalls mit einem zweiten, eine Injektionsflüssigkeit enthaltenden Behälter in einen Applikator eingesetzt werden. Durch zwischen den Containern angeordnete, im Hohlzylinder des Applikators verschiebbar gelagerte Träger mit einer doppelendigen Nadel zwischen den beiden Containern, kann eine Flüssigkeitsverbindung zwischen den ersten und zweiten Container hergestellt werden, sodass unter Wirkung eines ebenfalls in den Zylinderinnenraum des Applikators eingesetzten Kolbens die Flüssigkeit vom zweiten Behälter in den Wirkstoffbehälter eingepresst werden kann. Nach dem Einpressen der Injektionsflüssigkeit in den Wirkstoffbehälter kann durch weiteren Vorschub des Kolbens in den zylinderformigen Innenraum des Applikators eine Leitungsverbindung zu einem, mit einer Injektionsnadel verbindbaren Auslass hergestellt werden, sodass der rekonstituierte Wirkstoff nach der Rekonstitution unmittelbar verabreicht werden kann. Nachteilig bei diesem Applikator ist, dass elastisch verformbare Behälter benötigt werden und auch einige Nadeleinsätze erforderlich sind. Weiters ist es aus der DE 696 30 444 T2 bekannt, einen Applikatorteil als Aufnahmekammer für den Wirkstoff auszubilden, den Wirkstoff in diesem Applikatorteil zu lyophilisieren und im lyophilisiertern Zustand aufzubewahren.

Diese Aufnahmekammer ist an ihren abgabeseitigen, vorderen Gehäuseende mit einem abnehmbaren Verschluss mit einem Luer-Lock-Anschluss und an ihren vom vorderen Gehäuse abgewendeten Ende mit einem Kolben in Verbindung mit einem in diesem eingesetzten Adapter dicht verschlossen. Der Adapter ist zweiteilig ausgeführt und besteht aus zwei ineinander geführten, rohrförmigen Teilen, wobei der äußere Teil an der Innenwandung der Aufnahmekammer verschiebbar gelagert ist, wobei der innere rohrförmige Teil über ein Gewinde in den äußeren rohrförmigen Teil eingeschraubt ist und eine Durchtrittsöffnung im Kolben in die das den lyophilisierten Wirkstoff aufnehmenden Aufnahmekammer verschließt. Der innere Teil kann herausgeschraubt und anstelle dessen eine mit einem Lösungsmittel gefüllte Injektionsvorrichtung eingeschraubt werden. Durch Auspressen des Injektionsmittels aus dieser Injektionsvorrichtung in die Aufnahmekammer des lyophilisierten Werkstoffes kann dieser rekonstruiert werden, wobei der äußere Teil des Verschlussadapters durch den sich beim Einpressen des Lösungsmittels in der Aufnahmekammer des zu lyophilisierten Wirkstoffes in Richtung des offenen Endes der Aufnahmekammer zurückgeschoben wird und sie dann durch die Vorwärtsbewegung des Kolbens, nachdem dieser auf der der Aufnahmekammer zugewandten Stirnseite der Injektionsvorrichtung anliegt, der die Aufnahmekammer verschließenden Kolben nach Öffnen des am vorderen Gehäuse angeordneten Verschlusses und Einsetzen einer Injektionsnadel, die der in der Aufnahmekammer rekonstituierte Werkstoff wirkstoffentlüftet und der Wirkstoff darauf folgend injiziert werden kann. Nachteilig ist hierbei die Anwendung eines eigenen Verschlussadapters und die zeitaufwändige Handhabung zum Entfernen desselben und Einsetzen der Injektionsvorrichtung mit dem Lösungsmittel.

Weitere vorgefüllte Fertigspritzen mit Lyophilisat und Lösungsmittel, bei der die Lyophilisierungen der Spritze erfolgt, sind aus der US 6,440,101 B1 aus der DE 39 24 830 A1, aus der DE 196 38 940 A1, aus der DE 41 27 650 C1 und der DE 197 150 226 C bekannt.

Der vorliegenden Erfindung liegt die Aufgabe zugrunde eine Vorrichtung zum Lyophiliseren, Rekonstituieren und Verabreichen eines rekonstituierten Wirkstoffes zu schaffen, die eine sichere Aufbewahrung des lyophiliersten Wirkstoffes und eine sterile Aufbewahrung ermöglicht. Unabhängig davon soll sichergestellt werden, dass das Lyophilisat und der rekonstituierte Wirkstoff während des Mischvorganges und Verabreichens die Aufnahmekammer für den lyophilisierten Wirkstoff nicht verlässt.

Die Aufgabe der vorliegenden Erfindung wird durch die Merkmale in den unabhängigen Patentansprüchen 1 und 42 gelöst.

Vorteilhaft ist bei einer Ausführungsvariante nach Anspruch 1, dass die Vorrichtung in unterschiedlichen Vorbereitungsstufen jeweils verschlossen für den Einsatz aufbewahrt werden kann und trotzdem Einzelteile eines Gesamtsystems bilden, welches mit wenigen Handgriffen zu einer Injektionsvorrichtung bzw. zu einer Abgabevorrichtung zur Verabreichung des rekonstituierten Wirkstoffes zusammengesetzt werden kann. Vorteilhaft ist dabei, dass dadurch der gesamte Weg, den der Wirkstoff im Inneren der Vorrichtung nimmt ebenso wie der Weg des Lösungsmittels bzw. der Flüssigkeit zum Auflösen des Wirkstoffes steril ausgeführt ist und da dadurch die Verbindung zwischen der das Lyophilisat enthaltenden Kammer und der das Lösungsmittel enthaltenden Karpule erst bei Ingebrauchnahme geöffnet werden müssen und eine vorrangige Undichtheit von Verschlüssen daher möglichst vermieden ist. Weist die Vorrichtung jeweils den in der ersten Kammer aufbewahrten lyophilisierten Wirkstoff auf, so kann die Anzahl der auf Lager zu haltenden Vorrichtungen insgesamt reduziert werden, wenn die restlichen Vorrichtungsteile ebenfalls zusammengebaut und in einem sterilisierten Zustand abgepackt sind. Damit brauchen nur die Vorrichtungsteile mit dem Wirkstoff mit unterschiedlichen Wirkstoffen aufbewahrt werden und es kann eine geringere Anzahl von Betätigungsvorrichtung mit Karpule aufbewahrt werden, da diese mit den gleichartigen Vorrichtungsteilen, die unterschiedliche Wirkstoffe aufweisen, sofort gekuppelt werden können.

Besonders vorteilhaft ist es aber, wenn mit dem lyophilisierten Wirkstoff in der sterilisierten und versiegelten Vorrichtung bereits auch die Karpule mit der Flüssigkeit zum Rekonstituieren des lyophilisierten Wirkstoffes enthalten ist. Damit ist durch den verschlussseitigen Kolben, der die Flüssigkeit zum Rekonstituieren des Wirkstoffes aufweisenden Karpule, der Auden- und Innenbereich der Karpule sowie der Kammer für den Wirkstoff durchgehend sterilisiert und kann auch beim Zusammenbau mit den Antriebseinheiten beispielsweise einer Kolbenstange oder einem zusätzlichen Kolbenstangengehäuse bzw. einer Autoinjectoreinheit die Sterilität nicht zerstört werden.

Durch die Weiterbildung nach Anspruch 2 wird der Vorteil erreicht, dass ein entsprechendes Luftvolumen in der Kammer verbleibt, über dessen Verdichtung die Vorschubgeschwindigkeit oder die Einpressgeschwindigkeit der Flüssigkeit zum rekonstituieren des Wirkstoffes einfach gesteuert werden kann. Dazu kommt, dass es in einfacher Weise möglich ist, die Entlüftung des Innenraums der ersten Kammer beim Lyophilisieren des Wirkstoffes sicherzustellen und andererseits ohne großen Aufwand unmittelbar nach der Lyophilisierung des Wirkstoffes die erste Kammer ausreichend dicht und steril zu verschließen.

Vorteilhaft ist aber auch eine Ausgestaltung nach Anspruch 3, da dadurch das Volumen des Luftpolsters in der ersten Kammer einfach eingestellt werden kann.

Eine andere Weiterbildung ist nach Anspruch 4 vorgesehen. Die direkte Verwendung des rekonstituierten Wirkstoffes und die Rekonstituierung in der ersten Kammer werden durch die weitere Ausgestaltung nach Anspruch 4 erreicht.

Vorteilhaft ist auch eine Ausgestaltung nach den Ansprüchen 5 und 6, da durch die Vorbestimmung des Luftdruckes im Inneren der ersten Kammer kann die Vernischungszeit und die Einspritzgeschwindigkeit der Flüssigkeit in die erste Kammer beim Rekonstituieren des Wirkstoffes einfach eingestellt werden.

Eine weitere Ausführungsform nach Anspruch 7 hat sich insofern als vorteilhaft erwiesen, als dadurch mit einer einzigen Verbindungskanüle die Verbindung zwischen der das Lyophilisat enthaltenden Kammer und der das Lösungsmittel enthaltenden Karpule hergestellt wird, indem diese Verbindungskanüle den die Kammer verschließenden Kolben und die Dichtscheibe durchsticht, welche die Karpule verschließt.

Alternativ kann gemäß Anspruch 8 eine im Kolben aufgenommene Kanüle vorgesehen sein, wodurch ein die Kanüle tragendes Bauteil eingespart wird. Vorteilhaft ist dabei gemäß Anspruch 9 das gegen die Karpule gerichtete Kanülenende mit einer Kappe aus einem gummielastischen Material bedeckt, so dass auch bei dieser Ausführungsart die Sterilität im Inneren der Kammern gewährleistet ist.

Wenn nach Anspruch 10 das gegen die Karpule gerichtete Kanülenende in einer Vertiefung des Kolbens angeordnet ist und diese Vertiefung durch eine weitere Membrane verschlossen ist, die für Gas, jedoch nicht für Bakterien durchlässig ist, kann der die Kammer mit dem Lyophilisat enthaltende Teil der Vorrichtung bereits vor dem Zusammenfügen mit dem die Karpule enthaltenden teil steril gehalten werden.

Vorteilhaft kann gemäß Anspruch 11 die erste Kammer von einem Glaszylinder gebildet werden, der seinerseits im Gehäuse aufgenommen ist. In diesem Glaszylinder kann vorangehend der Wirkstoff lyophilisiert werden. Die Ausführungsart nach Anspruch 12 bietet Vorteile bei der Montage der Vorrichtung.

Die Ausführungsarten nach den Ansprüchen 13 und 14 erleichtern das sterile Verschließen und verhindern das unbeabsichtigte Öffnen der das Lyophilisat enthaltenden Kammer.

Die Ansprüche 15 und 16 betreffen Ausführungsarten, die beim Lyophilisieren des Wirkstoffes in der ersten Kammer vorteilhaft sind.

Vorteilhaft sind weiters Ausführungsarten nach den Ansprüchen 17 und 18, weil dabei sofort feststellbar ist, wenn an der Vorrichtung manipuliert wurde, was deren Sterilität beeinträchtigen könnte.

Die Ausführungsart gemäß Anspruch 19, welche ein zweiteiliges Gehäuse betrifft, bietet erhebliche Vorteile bei der Herstellung und Montage der Vorrichtung. Wenn nach Anspruch 20 die beiden Gehäuseteile durch ein Gewinde miteinander verbunden sind, lassen sie sich besonders einfach montieren. Auch diese Gewindeverbindung ist gemäß Anspruch 21 vorteilhaft mit einer Originalitätssicherung versehen, damit festgestellt werden kann, ob die Verbindung getrennt wurde.

Die Ausführungsarten nach den Ansprüchen 22 und 23 erleichtern das Lyophilisieren des Wirkstoffes in der ersten Kammer unter Aufrechterhaltung der Sterilität.

Die im Anspruch 24 beschriebene Ausführungsart ermöglicht die sterile Lagerhaltung der Vorrichtung ohne Betätigungsmittel. Zum Montieren der Betätigungsmittel dient vorteilhaft ein Kupplungsteil nach Anspruch 25.

Bei einer alternativen Ausführungsart gemäß den Ansprüchen 26 und 27 sind die Betätigungsmittel bereits montiert und in einem Schutzrohr aufgenommen, wodurch wiederum die Sterilität der ganzen Vorrichtung gewährleistet wird.

Vorteilhaft ist aber auch eine Weiterbildung der Vorrichtung nach dem Anspruch 28. Diese Lösung sichert neben einer raschen sicheren Kupplung der Kolbenstange zur Betätigung des Gesamtsystems auch gleichzeitig die Einmalverwendbarkeit der Vorrichtung ab. Zusätzlich wird dadurch aber auch die Möglichkeit eröffnet, die Flüssigkeitsrekonstitution des Wirkstoffes in die erste Kammer gegen einen entsprechenden Überdruck einzuführen, wobei gleichzeitig das Risiko ausgeschaltet wird, dass eine entgegen der Vorschubrichtung des Kolbens wirkende Rückstellkraft zu einem Eintritt von rekonstituierten Wirkstoff und zu einem Austritt in den Transferset bzw. in die Karpule aufgeschlossen wird.

Die bevorzugte Ausführungsart nach Anspruch 29 ermöglicht eine stufenweise Betätigung der Vorrichtung und die Ausführungsaft nach Anspruch 30 zeigt eine mögliche Ausgestaltung der Rastmittel.

Bei der Ausführungsart nach Anspruch 31 ist besonders vorteilhaft, dass durch die elastische Verformbarkeit der Kolhenstangenbereiche eine Bremswirkung erzielt wird, die ein zu rasches Vorschieben der Kolbenstange und damit eine unerwünschte Schaumbildung beim Mischen des Wirkstoffes mit dem Lösungsmittel verhindert.

Vorteilhaft ist aber auch eine Weiterbildung der Vorrichtung nach dem Anspruch 32. Durch diese Lösung wird eine bestimmte Abfolge der im Inneren der Vorrichtung während deren Betätigung ablaufenden Schritte sicher gestellt.

Die im Anspruch 33 definierte Ausführungsart dieser Vorrichtung zeigt eine besonders einfache Möglichkeit zur Realisierung der Rückhaltevorrichtung.

Vorteilhaft ist aber auch eine Weiterbildung der Vorrichtung nach dem Anspruch 34 gelöst Durch diese Lösung wird eine bestimmte Abfolge der im Inneren der Vorrichtung während deren Betätigung ablaufenden Schritte sicher gestellt.

Die besonderen Ausführungsarten nach den Ansprüchen 35 bis 37 zeigen Möglichkeiten einer einfachen und kostengünstigen Realisierung der Rückhaltevorrichtung des Kolbens.

Vorteilhaft ist aber auch eine andere Weiterbildung der Vorrichtung nach dem Anspruch 38. Die Merkmalskombination dieses Anspruchs definiert in überraschender und sehr vorteilhafter Weise eine Vorrichtung, bei welcher die Rekunstituierung des Wirkstoffes automatisch abläuft.

Weiters ist aber auch eine Vorrichtung nach dem Anspruch 39 vorteilhaft. Durch die Rückhaltevorrichtung wird die Sicherheit bei der Abfolge der während der Betätigung der Vorrichtung ablaufenden Vorgänge erhöht.

Die Ausfübrungsarten nach den Ansprüchen 40 und 41 zeigen vorteilhafte und sicher funktionierende Varianten der Rückhaltevorrichtung auf.

Die Aufgabe der Erfindung kann im weiteren auch unabhängig von der Ausbildung der Vorrichtung durch das Verfahren gemäß Anspruch 42 gelöst werden. Durch die gewählten Verfahrensabläufe wird nicht nur die Lyophilisierung des Wirkstoffes vereinfacht, sondern es wird ein durchgängiges Verfahren zur Herstellung des lyophilisierten Wirkstoffes und dessen steriler Verpackung zur wirtschaftlichen Weiterverwendung und einfachen Bedienung zur Abgabe des lyophilisierten, rekonstituierten Wirkstoffes erzielt. Außerdem kann dadurch eine hervorragende Qualität des lyophilisierten Wirkstoffes und dessen Ausreichende Lagerdauer erzielt werden, während gleichzeitig die Sterilität bis zum Rekonstituieren des Wirkstoffes sichergestellt ist. Ein Vorteil dieser Lösung liegt auch in den vielfältigen Möglichkeiten für die einzelnen Verfahrensschritte, die eine Mehrzahl von Vorrichtungstechnischen Möglichkeiten eröffnen und vor allem ein kostengünstiges Baukastensystem ermöglichen.

Die Erfindung wird im nachfolgenden anhand der in den Zeichnungen dargestellten Ausführungsbeispiele näher erläutert.

Es zeigen:
- Fig. 1: einen Längsschnitt durch eine erste Ausführungsart der Vorrichtung;
- Fig. 2: einen Längsschnitt durch eine zweite Ausführungsart der Vorrichtung;
- Fig. 3: einen Längsschnitt durch eine dritte Ausführungsart der Vorrichtung;
- Fig. 4a bis 4f: den Funktionsablauf beim Gebrauch der Vorrichtung nach Figur 2;
- Fig. 5a bis 5f: den Funktionsablauf beim Gebrauch der Vorrichtung nach Figur 3;
- Fig. 6: einen Längsschnitt durch eine Ausführungsart der Injektionseinheit;
- Fig. 7: einen Längsschnitt durch eine weitere Ausführungsart der Vorrichtung;
- Fig. 8a bis 8c: einen Längsschnitt durch eine Ausführungsart der Vorrichtung mit automatischer Aktivierung;
- Fig. 9a bis 9e: den Funktionsablauf bei einer weiteren Vorrichtung.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Die Ausführungsbeispiele zeigen mögliche Ausführungsvarianten der Vorrichtung, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvarianten möglich sind, vom Schutzumfang mit umfasst.

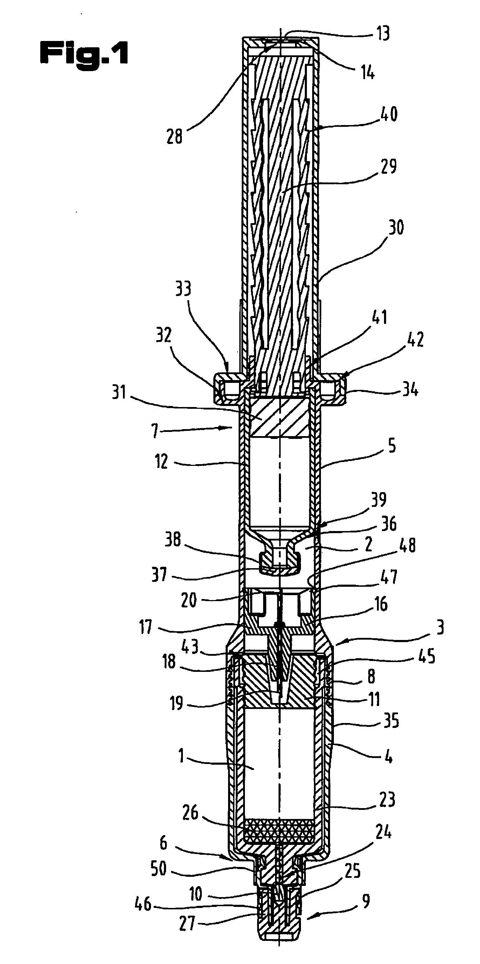

Figur 1 zeigt eine erste Ausführungsart einer erfindungsgemäßen Vorrichtung. Eine ein Lyophilisat 26 enthaltende erste Kammer 1 ist in einem Gehäuse 3 nahe bei dessen vorderem, abgabeseitigem Gehäuseende 6 angeordnet. Bei der Ausführungsart gemäß Figur 1 ist die erste Kammer 1 in einem Glaszylinder 23 gebildet, der seinerseits in dem aus Kunststoff bestehenden Gehäuse 3 untergebracht ist. Das Lyophilisat 26 wurde in der ersten Kammer 1 gebildet, wie dies später noch beschrieben wird.

Der das Lyophilisat 26 enthaltende Glaszylinder 23 hat an seinem nahe dem abgabeseitigen, vorderen Gehäuseende 6 gelegenen Ende eine verengte Mündung 24, auf die ein Adapter 25 aus Kunststoff aufgesetzt ist. Der Adapter 25 aus Kunststoff kann am Glaszylinder 23 angeformt bzw. angeklebt sein oder mit einer Schnappvorrichtung am Glaszylinder 23 befestigt sein.

Zur Befestigung des Adapters 25 auf einen, die abgangsseitige Stirnseite des Glaszylinders vorragenden zylinderförmigen Ansatzes, der durch die Mündung 24 besetzt ist, weist dieser Fortsatz zwischen der Stirnseite des Glaszylinders 23 und abgabenseitigen Ende desselben eine umlaufende Nut 50 auf. Von dem an der Stirnseite anliegenden Teil des Adapters 25 erstrecken sich in radialer Richtung elastische Schnapparme, die zur Längsmittelachse des Glaszylinders vorragende Schnapphaken aufweisen, in diese Nut 50 ein und wird damit der Adapter bewegungsfest mit dem Glaszylinder 23 verbunden. Der Adapter 25 weist weiters in seinen von dem Glaszylinder 23 abgewendeten Endbereich einen Luer-Lock-Ansatz auf. Selbstverständlich ist es auch möglich, einen Luer-Lock-Ansatz auszuformen, der mit entsprechenden Arretiermitteln zum Festsetzen und Halten des Verschlusses 9 bzw. einer, an den Adapter 25 anzuschließenden Injektionsnadel versehen sein kann.

Selbstverständlich können auch alle anderen, in der Medizintechnik eingesetzten Verschlussarten und Anschlussvorrichtung im Bereich dieses Adapters 25 ausgebildet sein.

Wie bereits vorher erwähnt, ist die kontaminationsfreie Applikation der Injektionslösung, d.h. des rekonstituierten Wirkstoffes bzw. des Lyophilisats 26, ein wesentlicher Vorteil der erfmdungsgemäßen Vorrichtung des erfindungsgemäßen Verfahrens. Diese Kontaminationsfreiheit muss über den gesamten Anwendungszeitraum der Vorrichtung gewährleistet sein. Für die Erfüllung bzw. Erzielung dieser Vorteile ist es die Realität des gesamten Innenraums der Vorrichtung. Um dies sicherzustellen, erfolgt die Abdichtung auf dem abgabeseitigen Gehäuseende 6 mittels der verrastbaren und bedarfsweise wieder lösbaren Verschlusskappe 27 mit dem integrierten Dichtelement 10 aus Pharmagummi, welches die Öffnung des Gaszylinders 23 bakteriendicht verschließt. Hierbei ist es vorteilhaft, wenn der Adapter 25 einen Luer-Lock-Verschluss mit Adaptierung aufweist, da dadurch auch beim Hantieren mit der Vorrichtung zu deren endgültigen Verwendung ein unbeabsichtigtes Lösen des Verschlusses 9 vermieden wird.

Auf dem Adapter 25 sitzt ein Verschluss 9, der aus einer Verschlusskappe 27 aus Kunststoff und einem in dieser gehaltenen Dichtelement 10 besteht. Letzteres besteht aus Pharmagummi und ragt in die Mündung 24, um diese dicht zu verschließen. Die Verschlusskappe 27 ist zusätzlich zum oder anstelle der Luer-Lock-Verriegelung mit einem Arretierungsansatz 46 versehen, der ein unbeabsichtigtes Entfernen des Verschlusses 9 verhindert. Bei abgenommenem Verschluss 9 bildet der Adapter 25 eine Luer- bzw. eine Luer-Lock Kupplung, mit der ein Luer-Anschluss, eine Injektionsnadel oder ein zu einer solchen führender Schlauch oder ein sonstiger Bauteil verbindbar ist.

An seinem der Abgabeseite der Vorrichtung entgegengesetzten Ende weist der Glaszylinder 23 in axialer Richtung verlaufende Bypässe 45 auf, die dem Gasaustausch beim Lyophilisierungsprozess dienen, wie weiter unten noch erläutert wird. In diesem Ende des Glaszylinders 23 sitzt ein Zylinderstopfen 11, der ebenfalls aus Pharmagummi besteht und eine solche axiale Erstreckung hat, bzw. in unterschiedlichen Positionen einnehmen kann, sodass er entweder über die Länge der Bypässe 45 hinaus reicht oder in eine Position verschoben werden kann, in der der Zylinderstopfen 11 den Glaszylinder 23 dicht abschließt.

Auf der der ersten Kammer 1 abgewandten, hinteren Seite des Zylinderstopfens 11 beginnt eine zweite Kammer 2, in der eine aus Glas oder Kunststoff bestehende Karpule 12 aufgenommen ist, die ein Lösungsmittel enthält. Die Karpule 12 ist an ihrem abgabeseitigen Ende mittels einer aus Pharmagummi bestehenden Karpulen-Dichtscheibe 37 verschlossen, die durch eine Bördelkappe 38 an der Karpule 12 festgehalten wird.

Im hinteren, der Abgabeseite abgewandten Ende der Karpule 12 sitzt ein Karpulenstopfen 31, der den das Lösungsmittel enthaltenden Innenraum der Karpule dicht verschließt. Zwischen der Außenoberfläche der Karpule 12 und der Innenoberfläche des Gehäuses 3 ist mindestens ein in Längsrichtung des Gehäuses 3 verlaufender Gasdurchgang vorgesehen. Dieser Gasdurchgang kann durch ein Spiel zwischen dem Außendurchmesser der Karpule und dem Innendurchmesser des Gehäuses bestehen, oder es können feine Längsrippen oder Längsrillen bzw. über die Oberfläche verteilte Erhebungen oder eine entsprechende Rauung innen im Gehäuse oder außen an der Karpule vorgesehen sein. Der Gasdurchgang dient der weiter unten noch genauer beschriebenen Sterilisierung der Vorrichtung mit Ethylenoxyd.

Damit die Karpule 12 sich nicht ungewollt in Richtung zum vorderen Gehäuseende 6 verschiebt, ist an der Innenwand des Gehäuses 3 mindestens eine Karpulenauflage 39 angeordnet, auf welcher sich die Karpule 12 mit ihrer Karpulenschulter 36 abstützt. Vorzugsweise sind mehrere Karpulenauflagen 39 am Umfang des Gehäuses 3 verteilt angeordnet, die beispielsweise durch Erweichen und leichtes Eindrücken der Gehäusewand oder mit einem eigenen, in dem Gehäuseteil einsetzbaren Bauteil gebildet werden können.

Zwischen der Karpulen-Dichtscheibe 37 und dem Zylinderstopfen 11 ist ein Transferset 16 angeordnet, welches die Aufgabe hat, beim Gebrauch der Vorrichtung das in der Karpule 12 enthaltene Lösungsmittel zwecks Rekonstitution des Lyophilisats in die erste Kammer 1 zu leiten. Das Transferset 16 enthält eine durchgehende, in einer Kanülenführung 17 gehaltene Verbindungskanüle 18. Die Verbindungskanüle 18 ist bei dieser Ausführungsart offen und daher auch für Gas durchgängig, so dass beide Seiten des Transfersets 16 mit Ethylenoxyd sterilisiert werden können. Das Transferset 16 wird durch an seinem Umfang angeformte Federrastlappen 47, die in eine im hinteren Gehäuseteil 5 angeordnete Ringnut 48 eingreifen, in seiner axialen Position im Gehäuse 3 festgehalten.

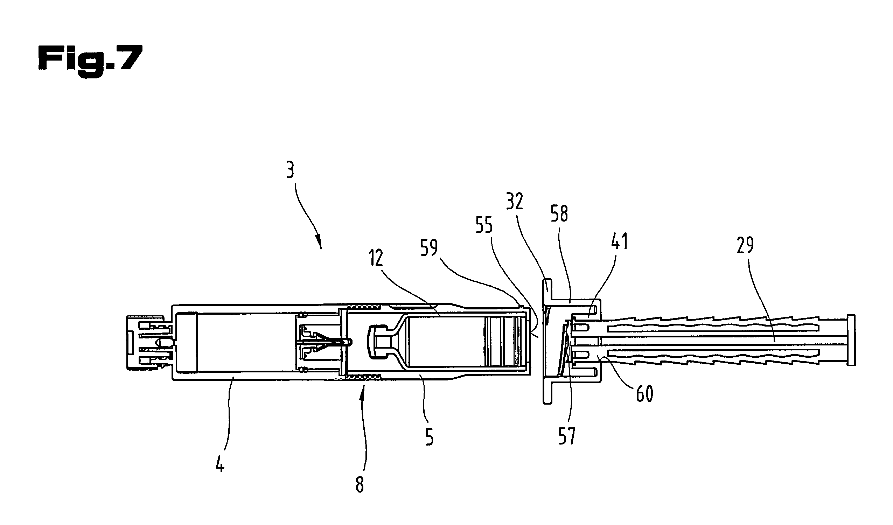

Das Gehäuse 3 der Vorrichtung kann zweckmäßigerweise aus einem vorderen, abgabeseitigen Gehäuseteil 4 und einem hinteren Gehäuseteil 5 bestehen, welche an einer Verbindungsstelle 8 miteinander lösbar verschraubt sind. In diesem Fall kann die Verbindungsstelle 8 mit einer Etikette 35 abgedeckt sein. Diese Etikette 35 kann zur Originalitätssicherung aber auch der Sicherstellung und Aufrechterhaltung der Sterilität des gesamten Innenraumes der Vorrichtung verwenden werden, weil die Zerstörung bzw. Bruch der Etikette 35 anzeigt, dass die Gehäuseteil 4 und 5 voneinander getrennt wurden und somit die Originalität und somit gegebenenfalls auch die Sterilität nicht mehr gegeben ist. Vorteilhaft ist es beispielsweise aber auch, wenn der hintere Gehäuseteil 5 mit einem senkrechten Ebenen zur Längsachse angeordneten Auflagefläche (Flansch 56 in Figur 7) zum Auflegen und Aufsiegeln einer Dichtfolie versehen ist

In diesem Fall reicht es, wenn in dem der Kolbenstange zugewandten Ende des hinteren Gehäuseteils 5 eine Verbindungsvorrichtung, insbesondere eine Schnappvorrichtung, angeordnet ist, mit der die Kolbenstange 29 bzw. eine erste von mehreren Rasten 40 zur Kupplung und Fixierung der Kolbenstange 29 dem hinteren Gehäuseteil 5 herangezogen werden kann.

Diese Verbindungsvorrichtung, die eine zur Längsachse springenden, umlaufenden Punkt gebildet sein kann, kann von radial zur Längsachse elastisch verstellbaren Schnapparmen hintergriffen werden und ermöglicht eine Halterung der Kolbenstange 29 zwischen dem Karpulenstopfen 31 und den einwärts ragenden Punkt der Verbindungsvorrichtung positioniert gehaltert ist und eine Vorschubbewegung auf den Karpulenstopfen 31 ausüben kann. Zur Positionierung der Karpule 12 in einem Abstand von das der im Karpulen-Dichtscheibe 37 in einem Abstand von der Spitze der Verbindungskanüle 18 des Transfersets 16 während des Transportes gehalten ist, kann die Karpule 12 mit einem partiell über die Oberfläche der Außenseite der Karpule 12 wirkenden Presssitz mit dem hinteren Gehäuseteil 5 verbunden sein, oder es sind entsprechende über die innere Oberfläche des hinteren Gehäuseteils 5 vor ragende Vorsprünge gebildete Halteelemente für die Karpule angeordnet. Dabei ist zu beachten, dass die Rückhaltekraft des Presssitzes bzw. der Rückhaltevorrichtungen geringer ist, als eine Halterung der Karpulen-Dichtscheibe 37 auf mit der Bördelkappe 38, da der Vorschub der Karpule 12 zum Eingriff in einer Transfersitz 16 unter Verwendung des Mediums in der Karpule 11, aufgrund der Inkompressibilität der Flüssigkeit über den Karpulenstopfen 31 der Karpule 12 erfolgt. D.h. die Verankerung der Karpulen-Dichtscheibe 37 muss so fest sein, dass die zum Überwinden des Presssitzes bzw. der Rückhalterung eine erforderliche die Kolbenstange 29 und den Karpulenstopfen 31 ausgeübte Vorschubkraft auf die Karpule im Bereich der Karpulen-Dichtscheibe 37 führt, bevor die Karpulen-Dichtscheibe 37 durch die Verbindungskanüle 18 des Transfersets 16 durchstochen ist.

Die Kolbenstange 29 ist mit mehreren Rasten 40 ausgestattet, deren Funktion weiter unten noch eingehender erläutert wird. Außerdem ist am hinteren Gehäuseende 7 eine flanschartige Fingerauflage 32 angeformt. Die Kolbenstange ist in einem im hinteren Gehäuseteil 5 geformten Bremszylinderansatz 41 axial verschiebbar aufgenommen.

Ein die Kolbenstange 29 vollständig abdeckendes Kolbenstangengehäuse 30 ist mit einem Verbindungsflansch 33 mit dem hinteren Gehäuseteil 5 verbunden, beispielsweise durch eine Verschweißung 42, die durch Ultraschall hergestellt oder Verklebung oder Anformung sein kann und nur so stark dimensioniert ist, dass sie gleichsam eine Sollbruchstelle und damit ein zusätzliches Qualitätskriterium bildet, die es ermöglicht, das Kolbenstangengehäuse 30 vom Gehäuse 3 abzunehmen. Eine den Verbindungsflansch 33 und die Fingerauflage 32 bedeckende Etikette 34 dient als Garantiesiegel, dessen Bruch anzeigt, dass das Kolbenstangengehäuse 30 vom Gehäuse 3 entfernt wurde.

Stirnseitig an seinem hinteren Ende weist das Kolbenstangengehäuse 30 eine Öffnung 13 auf, die durch eine auf einem Auflagegitter 28 ruhende Membrane 14 verschlossen ist. Die Membrane 14 ist derart beschaffen, dass sie für Gas durchlässig ist, jedoch nicht für Bakterien. So genanntes medizinisches Sterilpapier erfüllt dieses Erfordernis. Die Membran 14 dient dazu ein unabsichtliches Durchstoßen des Teretpapiers während der Sterilisation bzw. während den nachfolgenden Transport bis zur Verwendung vermeiden.

Diese Membran 14 kann einteilig in das Kolbenstangengehäuse 30 eingeformt bzw. mitgespritzt sein und es aber ebenso möglich das Auflagegitter einzupressen, einzukleben, einzurasten oder sonstig zu befestigen bzw. durch ein Fließ ein Gewebe, Gewirke oder eine sonstige Membran oder einen Filter zu ersetzen.

Der vordere Gehäuseteil 4, der Glaszylinder 23, der den Glaszylinder 23 hinten dicht verschließende Zylinderstopfen 11 und der den Glaszylinder 23 vorne dicht verschließende Adapter 25 mit dem auf diesen aufgesetzten Verschluss 9 bilden eine Einheit, die in einem abgeschlossenen Prozess hergestellt und als solche verkauft und/oder der weiteren Verarbeitung zugeführt werden kann. Die Herstellung und Montage dieser Einheit erfolgen in entsprechenden Reinräumen, um eine möglichst partikelfreie Produktion schon vor der Sterilisation sicherzustellen. Die Pharmagummiteile bestehen aus einer für die Lyophilisierung geeigneten Gummi-Formulierung.

Die Vormontage dieser Einheit geht so vor sich, dass zuerst der Verschluss 9 zusammengebaut wird, indem das Dichtelement 10 mit einer Montagevorrichtung in die Verschlusskappe 27 eingebaut wird. Dann wird der Verschluss 9 mit dem Adapter 25 z.B. mittels Luer-Lock-System verbunden, wobei die Verschlusskappe 27 mit dem Arretierungsansatz 46 im Adapter 25 einrastet, so dass eine sichere, aber wieder lösbare, Sicherung gegen unbeabsichtigtes Lösen der beiden Teile voneinander gegeben ist.

Nun werden vier Ausführungsarten der Befüllung des Glaszylinders 23 mit einer Wirkstofflösung und der Lyophilisation dieses Wirkstoffs im Glaszylinder 23 beschrieben. Zur Vorbereitung werden Zylinderstopfen 11, welche standardmäßig bereits gewaschen, silikonisiert und sterilisiert angeliefert wurden, in Aufnahmemagazine einer Abfüllanlage eingebracht und zur Weiterverarbeitung deponiert. Alternativ können die Zylinderstopfen 11 in einer Standard-Abfüllanlage auch gewaschen, silikonisiert, sterilisiert und getrocknet werden. Nun werden Glaszylinder 23 in die Abfüllanlage eingebracht, gewaschen, silikonisiert und im Heißlufttunnel sterilisiert.

Nach der ersten, zweiten und dritten Ausführungsart der Befüllung und Lyophilisation werden die vorbehandelten sterilen Glaszylinder 23 - in einer nachfolgenden Montagestation - mit den vormontierten Verschlüssen 9 abgabeseitig verschlossen. Dabei rastet der Adapter 25 über eine Schnappverbindung am Hinterschnitt des Glaszylinderhalses 23 unlösbar ein. In der Folge wird die Mündung 24 des Glaszylinders 23 mit dem Dichtelement 10 des Verschlusses 9 bakteriendicht verschlossen. Die abgabeseitig steril verschlossenen Glaszylinder 23 werden in Aufnahmen stehend in die Abfüllanlage eingebracht und die Wirkstofflösung kann jetzt in die offenen Glaszylinder 23 dosiert werden.

Nach der ersten Ausführungsart wird in diesem Moment der Lyophilisierungsvorgang eingeleitet, wobei die Entlüftung beziehungsweise der Gasaustausch über die noch offenen Querschnitte des Glaszylinders 23 erfolgt. Unmittelbar nach dem Abschluss der Lyophilisierung werden die Zylinderstopfen 11 automatisch in die Glaszylinder 23 gedrückt, wodurch diese dicht verschlossen werden. Bei dieser Ausführungsart kann auf die Bypässe 45 im Glaszylinder verzichtet werden.

Nach der zweiten Ausführungsart der Befüllung und Lyophilisation werden die vorbereiteten, sterilen Zylinderstopfen 11 zugeführt und in die Glaszylinder 23 genau positioniert eingesetzt, und zwar nur so weit, dass die Bypässe 45 des Glaszylinders 23 offen bleiben, so dass ein Gasaustausch während der nachfolgenden Lyophilisierung über die offenen Querschritte der Bypässe 45 erfolgen kann. Jetzt kann der Lyophilisierungsvorgang eingeleitet werden, wobei die Entlüftung beziehungsweise der Gasaustausch über die noch offenen Querschnitte der Bypässe 45 erfolgt. Unmittelbar nach dem Abschluss der Lyophilisierung werden die Zylinderstopfen 11 automatisch auf Endposition in die Glaszylinder 23 gedrückt, wodurch die Bypässe 45 dicht verschlossen werden.

Nach der dritten Ausführungsart der Befüllung und Lyophilisation werden - wie bei der ersten Ausführungsart - Glaszylinder 23 ohne Bypässe 45 verwendet. Hier kommen wieder Zylinderstopfen 11 zum Einsatz, deren Durchmesser größer ist als der Innendurchmesser der Glaszylinder 23 und die im Bereich ihres Umfangs axial verlaufende Entlüftungsdurchgänge aufweisen. Zumindest im Bereich dieser Entlüftungsdurchgänge weist der Zylinderstopfen 11 eine darüber hinausgehenden größeren Durchmesser auf, so dass beim tieferen Einschieben desselben in die Kammer die Entlüftungsdurchgänge durch die stärkere Verdichtung dicht verschlossen werden. Der Querschnitt dieser Entlüftungsdurchgänge ist dabei so dimensioniert, dass er durch die elastische Verformung des Zylinderstopfens 11 verschlossen wird, wenn dieser ganz im Glaszylinder 23 aufgenommen ist. Diese Zylinderstopfen 11 werden zunächst zugeführt und in die Glaszylinder 23 genau positioniert eingesetzt, und zwar nur so weit, dass die Entlüftungsdurchgänge im Zylinderstopfen 11 offen bleiben, so dass ein Gasaustausch während der nachfolgenden Lyophilisierung über die offenen Querschritte der Entlüftungsdurchgänge erfolgen kann. Unmittelbar nach dem Abschluss der Lyophilisierung werden die Zylinderstopfen 11 automatisch auf Endposition in die Glaszylinder 23 gedrückt, wodurch die Entlüftungsdurchgänge zugequetscht und so dicht verschlossen werden.

Bei der vierten Ausführungsart der Befüllung und Lyophilisation werden die Glaszylinder 23 zuerst an ihrem der Mündung 24 abgewandten Seite mit den Zylinderstopfen 11 dicht verschlossen und dann in Aufnahmen mit nach oben gerichteter Mündung 24 stehend in die Abfüllanlage eingebracht. Danach wird die Wirkstofflösung durch die Mündung 24 in die Glaszylinder 23 dosiert eingefüllt. Bei der nachfolgenden Lyophilisierung erfolgt der Gasaustausch durch die offene Mündung 24 des Glaszylinders 23. Nach Abschluss der Lyophilisierung werden die Glaszylinder 23 mit den vormontierten Verschlüssen 9 abgabeseitig verschlossen. Dabei rastet der Adapter 25 über eine Schnappverbindung am Hinterschnitt des Glaszylinderhalses 23 unlösbar ein. In der Folge wird die Mündung 24 des Glaszylinders 23 mit dem Dichtelement 10 des Verschlusses 9 bakteriendicht verschlossen.

Gleichgültig nach welcher der vier Ausführungsarten die Glaszylinder 23 gefüllt und lyophilisiert wurden, können diese jetzt, da beidseitig bakteriendicht verschlossen, dem weiteren, gegebenenfalls externen Fertigmontageprozess zugeführt werden.

Die nachfolgend beschriebenen Montageschritte der Vorrichtung können zeitlich und örtlich unabhängig von den bisher beschriebenen Montageschritten durchgeführt werden. Der fertig gestellte, beidseitig bakteriendicht verschlossene Glaszylinder 23 mit dem lyophilisierten Wirkstoff wird mittels einer Montagevorrichtung in den vorderen Gehäuseteil 4 eingebracht. Dabei verrasten außen am Adapter 25 erhaben angebrachte Riefen mit am Mündungsansatz des vorderen Gehäuseteils 4 innen vertieft angebrachten Rillen. Dadurch ist eine sichere Kupplung und gegebenenfalls ein Verdrehungsschutz zwischen dem Gehäuseteil 4 und dem Adapter 25 und dem Glaszylinder 23 erzielt, sodass der Verschluss 9 von der Vorrichtung abgenommen werden kann, ohne dass es zu einer Relativbewegung oder gegebenenfalls einem Verdrehen zwischen dem Glaszylinder 23 relativ zum Adapter 25 bzw. dem Gehäuseteil 4 kommen kann. Die jetzt komplettierte Einheit, die im Folgenden als Injektionseinheit bezeichnet wird, ist damit zur weiteren Montage fertig gestellt.

Die nachfolgend beschriebenen Montageschritte der Vorrichtung können wiederum zeitlich und örtlich unabhängig von den bisher beschriebenen Montageschritten durchgeführt werden.

Eine weitere Einheit der Vorrichtung wird wie folgt gebildet. In den an seinem hinteren Ende z.B. durch den nach Innen umlaufenden Bund verengten hinteren Gehäuseteil 5 wird von der Verbindungsstelle 8 her zuerst die die Karpule 12 mit dem Lösungsmittel (Wasser für injizierbare Präparate) und schließlich das Transferset 16 eingeführt.

Am hinteren Ende des hinteren Gehäuseteils 5 wird durch die Verengung erst die Kolbenstange 29 eingeführt, bis diese mit ihrer Schnappverbindung einrastet, dann wird das Kolbenstangengehäuse 30 mit dem angeformten Verbindungsflansch 33 auf die Fingerauflage 32 des Gehäuseteils 5 aufgesetzt. Zusätzlich wird die Verbindung noch mit einer 4-PunktSchweißung gesichert. Anschließend wird die Flansch Fingerauflagenverbindung rundum etikettiert, um den Originalitätsnachweis erbringen zu können.

Diese, als Aktivatoreinheit bezeichnete Einheit kann jetzt mit der vorangehend beschriebenen Injektionseinheit verbunden werden, indem der hintere Gehäuseteil 5 auf den vorderen Gehäuseteil 4 geschraubt und die Verbindungsstelle 8 anschließend ebenfalls rundum etikettiert wird, um auch an dieser Stelle den Qualitätsnachweis erbringen zu können.

Die derart fertig montierte Vorrichtung kann nun einem abschließenden Sterilisationsprozess zugeführt werden, bei welchem ein Gas, vorzugsweise Ethylenoxyd, dem Innenraum der Vorrichtung zugeführt wird, ohne dabei in die erste Kammer 1 zu gelangen. Das Gas dringt dabei durch die Membrane 14 ein, mit der die im hinteren Ende des Kolbenstangengehäuses 30 vorhandene Öffnung 13 bakteriendicht abgedeckt ist, gelangt zunächst ins Innere des Kolbenstangengehäuses, wobei es diesen Innenraum mitsamt der darin enthaltenen Kolbenstange 29 sterilisiert. Durch den weiter oben beschriebenen Gasdurchgang zwischen dem Gehäuseteil 5 und der Karpule 12 strömt das Gas weiter zum abgabeseitigen Ende der Karpule 12 und weiter durch die Transferkanüle 18 bis zum hinteren Ende des Zylinderstopfens 11, wobei es alle Flächen sterilisiert, mit denen es in Kontakt kommt. Es liegt auf der Hand, dass eine solcherart aufgebaute und sterilisierte Vorrichtung für die Lagerung und den Versand nicht extra steril verpackt werden muss.

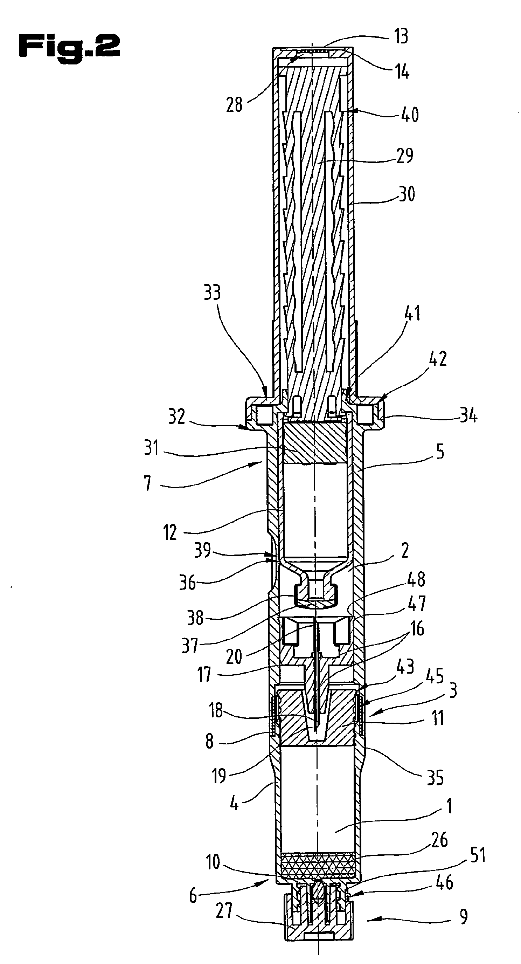

In Figur 2 ist eine weitere Ausführungsart der Vorrichtung dargestellt. Der wesentlichste Unterschied zur Ausführungsart gemäß Figur 1 besteht darin, dass die erste Kammer nicht in einem Glaszylinder gebildet ist, sondern direkt in dem aus Kunststoff bestehenden vorderen Gehäuseteil 4. Zur Befestigung des Verschlusses 9 ist ein Kupplungsansatz 51, der vorzugsweise als Luer-Lock-Ansatz ausgebildet ist, direkt am vorderen Gehäuseteil 4 angeformt. Auf der gegenüberliegenden Seite ist die erste Kammer 1, wie bei der Ausführungsart gemäß Figur 1, mit einem Zylinderstopfen 11 aus Pharmagummi bakteriendicht verschlossen, wobei auch hier zwei verschiedene Ausführungsarten des Zylinderstopfens 11 eingesetzt werden können, nämlich mit oder ohne Entlüftungsdurchgänge. In dem in Figur 2 dargestellten Ausführungsbeispiel sind Bypässe 45 im vorderen Gehäuseteil 4 vorhanden und der Zylinderstopfen 11 weist dementsprechend keine Entlüftungsdurchgänge auf.

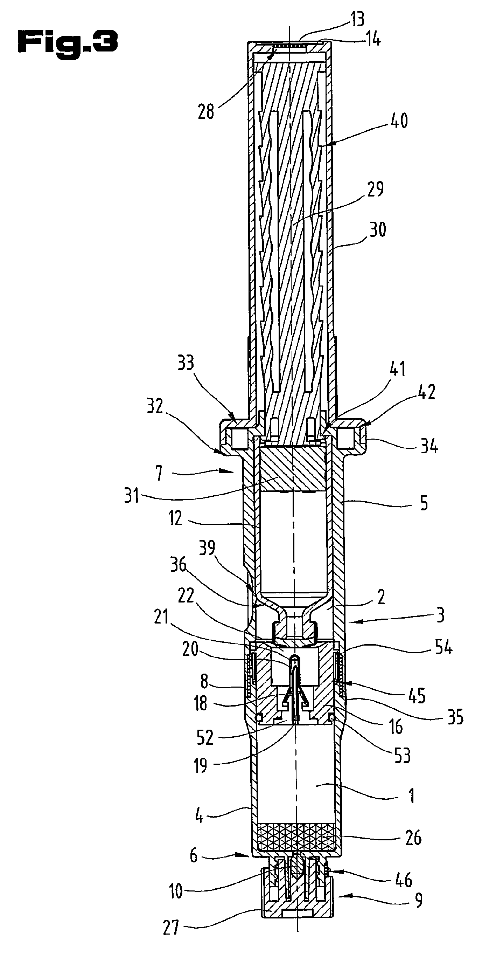

Die Ausführungsart der Vorrichtung gemäß Figur 3 ist ähnlich aufgebaut wie die vorangehend beschriebene Ausführungsart gemäß Figur 2. Auch hier ist die erste Kammer 1 in dem aus Kunststoff hergestellten vorderen Gehäuseteil 4 gebildet. Jedoch ist bei dieser Ausführungsart eine andere Ausführungsart des Transfersets 16 eingebaut, die so aufgebaut ist, dass das Transferset 16 die hintere Begrenzung und Abdichtung der ersten Kammer 1, wie auch die Funktion eines Kolbens zum Ausstoßen des rekonstituierten Wirkstoffes aus der ersten Kammer 1 übernimmt. Folglich ist bei dieser Ausführungsart der im Zusammenhang mit den Ausführungsarten nach den Figuren 1 und 2 beschriebene Zylinderstopfen 11 überflüssig. Das Transferset 16 gemäß Figur 3 weist eine Verbindungskanülenaufnahme 52 auf, in welcher die Verbindungskanüle 18 gehalten ist und welche gleichzeitig zum dichten Aufnehmen einer das karpulenseitige Kanülenende 20 überdeckenden Kanülenschutzkappe 21 dient. Anders als beim Transferset 16 nach den Ausführungsarten gemäß Figur 1 und 2 ragt bei dieser Ausführungsart das abgabeseitige Kanülenende 19 nicht über das Transferset hinaus. Zur Abdichtung des Transfersets 16 im vorderen Gehäuseteil 4 ist bei dieser Ausführungsart ein O-Ring 53 vorgesehen. Federrastlappen 47 übernehmen die Punktion des Bremswulstes 43, der bei den Ausführungsarten nach Figur 1 und 2 am Zylinderstopfen 11 vorgesehen ist.

Die weiter vorne im Zusammenhang mit der Ausführungsart nach Figur 1 beschriebenen Varianten der Befüllung und Lyophilisation sind auch bei den Ausführungsarten gemäß den Figuren 2 und 3 anwendbar. Der einzige Unterschied besteht darin, dass an Stelle des Glaszylinders 23 bei diesen Ausführungsarten der Vorrichtung der vordere Gehäuseteil 4 mit dem Wirkstoff befüllt und der Wirkstoff darin lyophilisiert wird.

Die Auswahl der für die vorangehend beschriebenen Ausführungsarten der Vorrichtung eingesetzten Werkstoffe erfolgt primär mit dem Ziel der langfristigen Sicherstellung von Funktionalität und mechanischer Stabilität, auch unter extremen Bedingungen. Für die Karpule 12, den vorderen Gehäuseteil 4, den Zylinderstopfen 11, den Karpulenstopfen 31, die Karpulen-Dichtscheibe 37, die Verbindungskanüle 18, die Injektionskanüle 49 sowie alle Dichtelemente wie O-Ringe ist darüber hinaus die Erfüllung der Anforderungen an Materialien zur Herstellung von Medizinprodukten erforderlich. Mit Rücksicht auf die notwendige Sterilisation ist für alle Bauteile neben der Erfüllung der physikalischen Anforderungen auch die Beständigkeit der eingesetzten Werkstoff gegenüber Ethylenoxid von Bedeutung. Aus diesen Gründen wird der vordere Gehäuseteil vorzugsweise aus Cyclolefin Copolymer, im Folgenden COC genannt, hergestellt. Es handelt sich dabei um einen amorphen Thermoplast, der unter dem Handelsnamen Topas 6013 bekannt ist. Der vordere Gehäuseteil 4 übernimmt bei den Ausführungsarten nach Figur 2 und 3 die Funktion einer Primärverpackung, welche das Lyophilisat beinhaltet. Die Wahl des genannten Werkstoffes für die Ausführung dieses Gehäuseteils erfolgte daher im Hinblick auf die hohen Anforderungen an die präzise Formgebung, chemische Resistenz, glasartige Transparenz, große Wärmeformbeständigkeit und Bruchfestigkeit, Autoklavierbarkeit bei 121°C sowie Sterilisierbarkeit durch Gammabestrahlung und Ethylenoxid. Weiters erfüllt COC, aufgrund der hohen Materialreinheit und Inertheit alle Anforderungen, welche an Pharmabehälter für die Aufbewahrung von Parenteralien gemäß EP/JP und USP inkl. USP Class VI., an diese gestellt werden. Durch die hohen Verarbeitungstemperaturen kann außerdem Pyrogenfreiheit garantiert werden. Weiters ist es durch die hervorragende Barriere gegen Wasserdampf möglich, Medikamente über mehrere Jahre in Behältern zu lagern. Die Behälter aus COC eignen sich, aufgrund der oben angegebenen Materialeigenschaften, besonders für die Verpackung von biotechnischen Produkten, toxischen Produkten, Lösungsmitteln, Lyophilisaten und anderen pharmazeutischen Präparaten, so dass Pharmabehälter aus Kunststoff eine echte Alternative zu Pharmabehälter aus Glas geworden sind. Weiters kann durch die formtechnischen Gestaltungsmöglichkeiten, welche durch den Einsatz von Thermoplasten möglich sind, die Form des vorderen Gehäuseteils 4 entsprechend den Anforderungen an Größe, Entlüftungskanäle (Bypässe) Spritzenkopf und Hals usw. optimal gestaltet und hergestellt werden.

Die Karpule 12 und die erste Kammer 1 nach dem Ausführungsbeispiel nach Figur 1, also der Glaszylinder 23 werden bevorzugt aus Borsilicatglas hergestellt, welches beispielsweise unter dem Handelsnamen Fiolax bekannt ist. Dieser Glastyp entspricht den Anforderungen der Arzneibücher USP, Europäische Pharmakopöe, Japanische Pharmakopöe und dem deutschen Arzneibuch (DAB) hinsichtlich chemischer Beständigkeit und Lichtschutz. Die Einhaltung der Schwermetallgrenzwerte (Blei, Cadmium, Quecksilber, Chrom VI) gemäß der europäischen Richtlinie 94/62/CE ist gewährleistet. Glas ist gegenüber Ethylenoxid völlig inert und dicht.

Für die Kanülenführung 17 kommt aus Gründen der mechanischen Beanspruchung (Federwirkung der Federrastlappen 47) ein Polyacetat (POM) zu Einsatz. POM ist vom Hersteller auch für die Herstellung von Teilen zur Verwendung im Lebensmittelkontakt konzipiert und entspricht den Bestimmungen der FDA und der Europäischen Union. Analog zur Kolbenstange 29 gilt jedoch auch für die Kanülenfürhrung, dass sie bei bestimmungsgemäßem Gebrauch der Vorrichtung keinen direkten Kontakt zum Arzneimittel und dem menschlichen Körper aufweist. Da die Kanülenführung die Verbindungskanüle 18 aufnimmt, wurde trotzdem Wert darauf gelegt, ein Material, welches vom Hersteller für die Anwendung im Bereich von Medizinprodukten spezifiziert ist, einzusetzen.

Für die Verbindungskanüle 18 wurde Edelstahl (NIRO 1.403, AISI 304) gewählt. Der eingesetzte Edelstahl ist für die Herstellung von medizinischen Kanülen geeignet und spezifiziert und auch für die Sterilisation mit Ethylenoxid geeignet. Die Fixierung der Verbindungskanüle 18 in der Kanülenführung 17 erfolgt mit Hilfe eines UV-härtenden Cyano-Acrylatklebers, beispielsweise eines unter dem Handelsnamen Loctite 4304 bekannten Klebers. Die Überprüfung der Klebereignung zur Sicherstellung der Kanülenfixierung auch nach der Sterilisation erfolgte in Form der Kanülenfestsitzprüfung (Festigkeit zwischen Kanülenführung 17 und Verbindungskanüle 18) gemäß EN ISO 7864 an den Injektionseinheiten. Die Gegenüberstellung der Untersuchungsergebnisse für die Nadelausziehkraft unsterilisierter und sterilisierter Proben zeigt keine nachteilige Veränderung durch die Sterilisation. Die an den sterilisierten Proben ermittelten Werte für die Ausziehkraft erfüllen nicht nur die Normanforderungen gemäß EN ISO 7864, sondern liegen sogar deutlich über den Werten der unsterilisierten Proben.

Der gewählte Sterilisationsprozess mit Ethylenoxid dient zur Sicherstellung der Keimfreiheit der gesamten Vorrichtung, ausschließlich des darin enthaltenen, in der ersten Kammer 1 gasdicht verpackten Arzneimittels (Lyophilisat) und des in der Karpule 12 gasdicht verpackten Lösungsmittels (WFI). Die als bakteriendichte Barriere fungierenden Membranen 14 und 15 sind aus medizinischem Papier mit einem Flächengewicht von 60g/m² gefertigt. Das Papier ist zwecks Siegelbarkeit mit dem Kolbenstangengehäuse 30 mit Polyethylen beschichtet. Das verwendete Material ist für den Einsatz im medizinischen/pharmazeutischen Bereich, speziell für die Sterilgutversorgung konzipiert und entspricht den Anforderungen gemäß EN 868 Teil 1 und 7. Es ist gut durchlässig für Ethylenoxid und als bakteriendicht spezifiziert.

Die ETO-Sterilisation der Vorrichtung darf zu keinerlei Veränderung des in der bakteriendicht verschlossenen ersten Kammer 1 befindlichen Lyophilisates und des in der Karpule 12 befindlichen Lösungsmittel, führen. Um dies sicherzustellen, muss gewährleistet sein, dass kein Ethylenoxid in den Innenraum der ersten Kammer 1 und der Karpule 12 eindringen kann. Die praktische Überprüfung der Ethylenoxiddichtheit des Spritzenzylinders mit dem Lyophilisat und der Karpule mit dem Lösungsmittel (WFI) erfolgte durch die Untersuchung des Karpuleninhalts und des Inhalts der ersten Kammer 1 sterilisierter erfindungsgemäßer Vorrichtungen, in den beschriebenen Ausführungsformen auf Ethylenoxid. In keiner der mit Ethylenoxid sterilisierten Karpulen und ersten Kammern konnte Ethylenoxid nachgewiesen werden. Somit kann eine Veränderung des darin gelagerten Lösungsmittels (Wasser für Injektionszwecke) und des Lyophilisates durch die Einwirkung von Ethylenoxid ausgeschlossen werden.

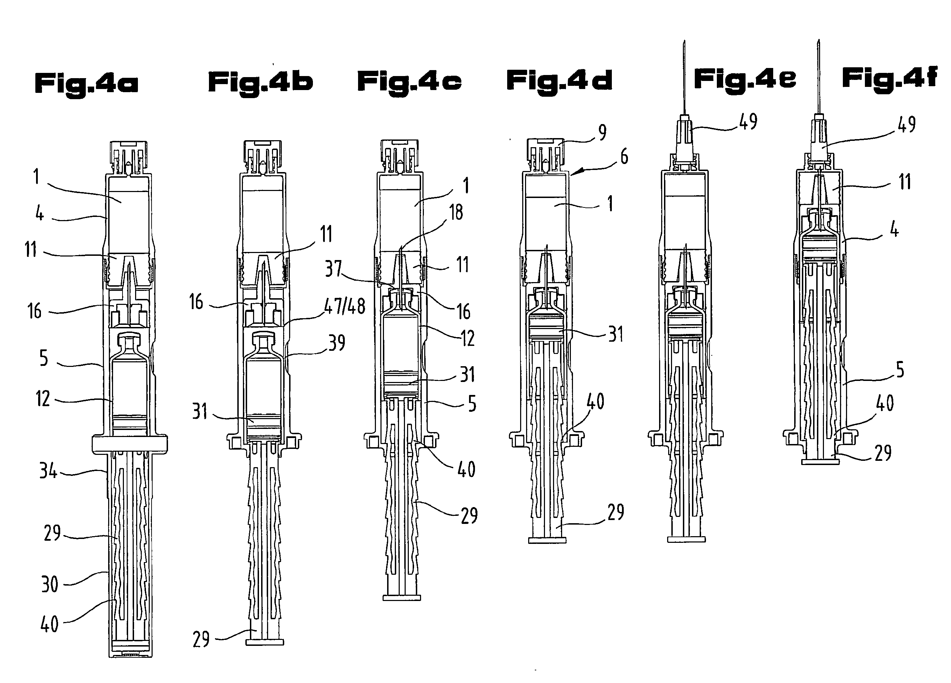

Nachstehend wird unter Bezugnahme auf die Figuren 4a bis 4f die Benutzung der beschriebenen Vorrichtung in der Ausführungsart gemäß Figur 2 erläutert.

In der in Fig. 4a gezeigten Ausgangsposition ist die Aktivatoreinheit mit dem Kolbenstangengehäuse 30 gesichert und die Verbindung zwischen der Aktivatoreinheit und der Injektionseinheit (hinterer Gehäuseteil 5) mit der Etikette 34 versehen.

In der Position gemäß Fig. 4b ist das Kolbenstangengehäuse 30 vom hinteren Gehäuseteil 5 abgenommen und die Vorrichtung kann jetzt, wie nachfolgend beschrieben, aktiviert werden.

In der Position nach Fig. 4c wird durch leichten Druck auf die Kolbenstange 29 die Vorrichtung durch die Kolbenstangenbewegung aktiviert. Dieser Druck wird auf den Karpulenstopfen 31 übertragen. Da die Karpule dicht verschlossen und mit einem flüssigen Lösungsmittel gefüllt ist, kann der Karpulenstopfen 31 nicht in die Karpule 12 hinein gedrückt werden. Folglich löst sich die Karpule 12 gegen den Widerstand der Karpulenauflage 39 aus der Ruheposition und bewegt sich in Richtung Transferset 16. In der Folge lösen sich die Federrastlappen 47 des Transfersets 16 aus der Ringnut 48 des Gehäuseteils 5 und das Transferset 16 wird Richtung Zylinderstopfen 11 gedrückt, bis dieses am Zylinderstopfen 11 aufsitzt. Dabei durchsticht die im Transferset 16 integrierte Verbindungskanüle 18 den Zylinderstopfen 11 und dringt in den Innenraum der ersten Kammer 1 ein. In der Folge durchsticht das andere Ende der Verbindungskanüle 18 auch die Karpulen-Dichtscheibe 37 der Karpule 12 und die Verbindungskanüle 18 dringt in den Innenraum der Karpule 12 ein, sodass jetzt eine durchgehende Verbindung zwischen dem Karpulen-Innenraum (mit dem Lösungsmittel) und der ersten Kammer 1 (mit dem Lyophilisat) hergestellt ist.

Eine der Rasten 40 der Kolbenstange 29 rastet jetzt automatisch im hinteren Gehäuseteil 5 ein und sichert das Transferset 16 in Verbindung mit dem Zylinderstopfen 11 in dieser Position ab. Ein Zurückziehen der Kolbenstange aus dieser Position ist nicht mehr möglich. Dadurch ist sichergestellt, dass die Verbindung zwischen der ersten Kammer 1 mit dem Lyophilisat und der Karpule mit dem Lösungsmittel über die Verbindungskanüle 18 aufrecht bleibt.

Durch die weiterführende Kolbenstangenbewegung von der in Fig. 4c dargestellten in die in Fig. 4d dargestellte Position wird durch den Druck auf den Karpulenstopfen 31 das Lösungsmittel aus der Karpule 12 durch die Verbindungskanüle 18 in die erste Kammer 1 überführt und damit das Lyophilisat langsam aufgelöst. Der beim Überführen des Lösungsmittels entstehende Gegendruck, welcher sich durch das Zusammendrücken des Luftpolsters in der ersten Kammer 1 aufbaut, bewirkt einen Bremseffekt, so dass das Lösungsmittel nicht zu schnell überführt wird und ein übermäßiges Schäumen der Arzneimittellösung verhindert wird. Eine zusätzliche Bremswirkung wird durch den Widerstand der federnden Rasten 40 der Kolbenstange 29 gegen den Widerstand des Bremszylinderansatzes 41 im hinteren Gehäuseteil 5 erzielt. Eine weitere Raste 40 rastet im hinteren Gehäuseteil 5 ein und hält die Karpule 12 in Verbindung mit dem Transferset 16 gegen den aufgebauten Innendruck (Luftpolster) in der ersten Kammer 1 in dieser Position fest. Durch diese Sperre wird das Zurückfließen von Injektionslösung in die Karpule sicher unterbunden. Ist die Position gemäß Fig. 4d erreicht, wird die Vorrichtung leicht geschüttelt und dabei überprüft, ob das Lyophilisat vollständig aufgelöst ist. Danach kann der Verschluss 9 entfernt werden, wobei die Vorrichtung mit ihrem abgabeseitigen Ende 6 nach oben zu halten ist, so dass der in der ersten Kammer 1 vorhandene Luftdruck abgebaut wird, ohne dass Injektionslösung entweicht. Nach dem Aufsetzen einer Injektionskanüle 49 und Entlüften der ersten Kammer 1 über diese ist die Vorrichtung nun zur Injektion bereit. Diese Position ist in Fig. 4e dargestellt.

Während der Injektion wird die Injektionslösung mittels des Zylinderstopfens 11 aus der ersten Kammer 1 gedrückt. Nach erfolgter Injektion erreicht die Vorrichtung schließlich die in Fig. 4f gezeigte Position. Dabei rastet eine weitere Raste 40 im Gehäuseteil 5 ein und sichert die Kolbenstange 29 in ihrer Endposition gegen ein Herausziehen. Damit ist eine Wiederverwendung der Vorrichtung wirksam verhindert.

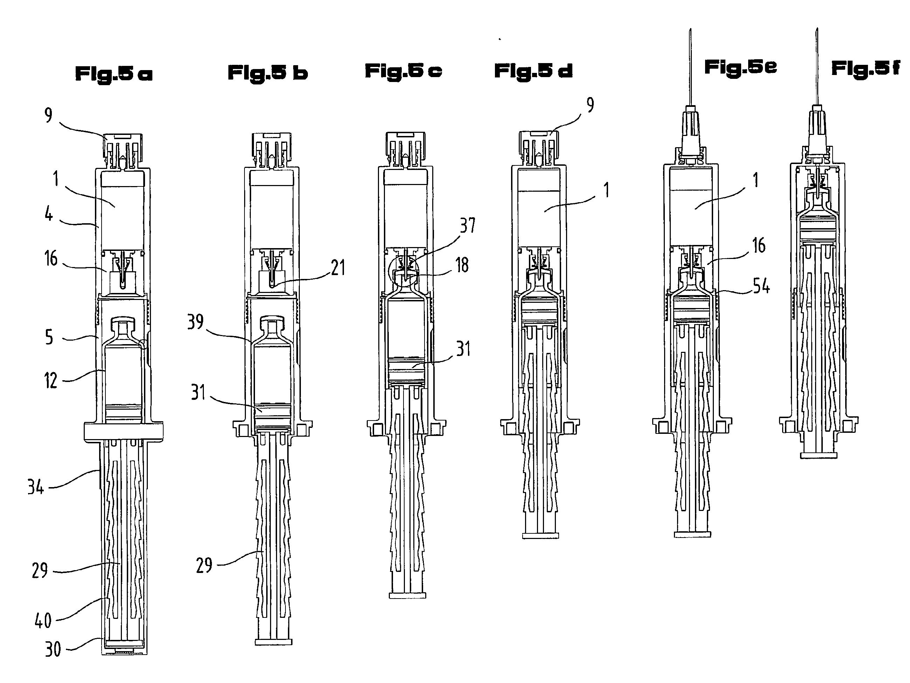

Die vorangehend anhand der Figuren 4a bis 4f im Zusammenhang mit einer Vorrichtung gemäß Figur 2 beschriebenen Abläufe spielen sich bei einer Vorrichtung nach Figur 1 genau gleich ab. Die Abläufe bei einer Vorrichtung nach Figur 3 werden nachfolgend anhand der Figur 5 beschrieben.

In der in Figur 5a dargestellten Ausgangsposition ist die Aktivatoreinheit mit dem Kolbenstangengehäuse 30 gesichert und die Verbindung zwischen Aktivatoreinheit und der Injektionseinheit (hinterer Gehäuseteil 5) mit dem Originalitätsverschluss (Etikette 34) versehen.

In der Position gemäß Figur 5b ist das Kolbenstangengehäuse 30 bereits vom hinteren Gehäuseteil 5 abgenommen und die Vorrichtung kann jetzt aktiviert werden. Durch leichten Druck auf die Kolbenstange 29 wird durch die Kolbenstangenbewegung das System aktiviert. Durch den Druck auf den Karpulenstopfen 31 löst sich die Karpule 12 gegen den Widerstand der Karpulenauflage 39 aus der Ruheposition und bewegt sich in Richtung Transfer-Set 16.

Während des Übergangs von der Position nach Figur 5b zur Position nach Figur 5c wird die Kanülenschutzkappe 21 zusammengedrückt, die sterile Verbindungskanüle 18 durchsticht die Karpulen-Dichtscheibe 37 und dringt in den Innenraum der Karpule 12 (mit dem Lösungsmittel) ein. Das Transfer-Set 16 bleibt dabei, gesichert durch die angeformten Bremsfahnen 54, auf Position stehen. Die Raste 40 der Kolbenstange 29 rastet jetzt automatisch im hinteren Gehäuseteil 5 ein und sichert das Transfer-Set 16, in Verbindung mit der Verbindungskanüle 18 und der Karpule 12, in der Position gemäß Figur 5c ab. Ein Zurückziehen der Kolbenstange aus dieser Position ist nicht mehr möglich.

Durch die weiterführende Kolbenstangenbewegung wird durch den Druck auf den Karpulenstopfen 31 das Lösungsmittel aus der Karpule 12 durch die Verbindungskanüle 18 in die erste Kammer 1 überführt und das Lyophilisat langsam aufgelöst. Nach dem Lösungsmittel-Transfer in die erste Kammer 1 rastet eine weitere Raste 40 der Kolbenstange 29 wieder automatisch im hinteren Gehäuseteil 5 ein und sichert die Karpule 12, in Verbindung mit dem Transfer-Set 12, in dieser Position gemäß Figur 5d ab. Durch diese Sperre ist ein Zurückfließen von Injektionslösung, durch den aufgebauten Druck in der ersten Kammer 1 nicht mehr möglich.

Nachdem das Lyophilisat vollständig rekonstituiert ist, wird der Verschluss 9 abgenommen und die Injektionskanüle 49 aufgesetzt (Figur 5e). Nach dem Entlüften der ersten Kammer 1 über die Kanüle kann die Injektion erfolgen. Dabei werden die Bremsfahnen 54, die das Transferset 16 bisher in einem Ansatz im vorderen Gehäuseteil 4 zurückgehalten haben, weggedrückt und das Transferset 16 beginnt, sich zur Abgabeseite der Vorrichtung hin zu bewegen. Das Transfer-Set 16 dient jetzt als Spritzenkolben und drängt die Injektionslösung aus der ersten Kammer 1.

Nach erfolgter Injektion erreicht die Vorrichtung schließlich die in Fig. 5f gezeigte Position, in welcher eine weitere Raste 40 im Gehäuseteil 5 einrastet und die Kolbenstange 29 in ihrer Endposition gegen ein Herausziehen sichert. Damit ist eine Wiederverwendung der Vorrichtung wirksam verhindert.

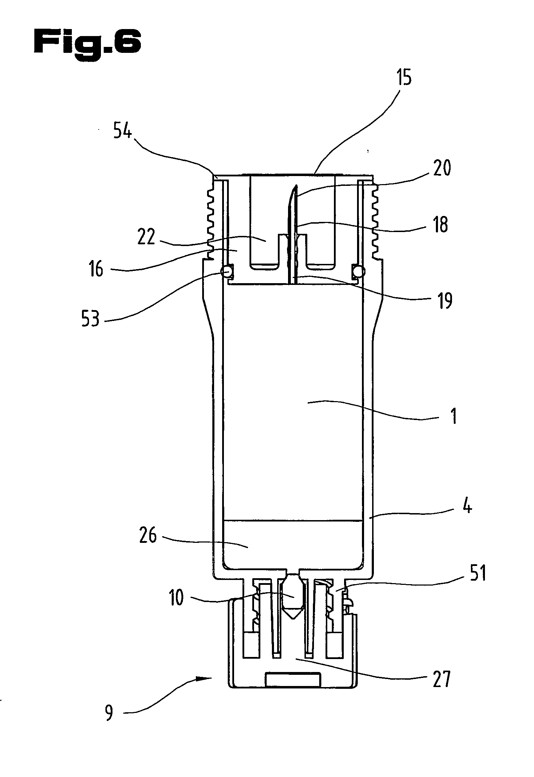

Figur 6 zeigt eine Ausführungsart einer Injektionseinheit, die separat hergestellt, gelagert und verkauft und später mit einem Aktivatorteil zusammengebaut werden kann. Wie bei den Ausführungsarten nach den Figuren 2 und 3 ist bei dieser Ausführungsart die erste, das Lyophilisat 26 enthaltende Kammer 1 direkt durch den vorderen Gehäuseteil 4 gebildet und weist abgabeseitig einen Kupplungsansatz 51 auf, der einen Luer-Lock-Ansatz bildet und den aus der Verschlusskappe 27 mit dem darin angeordneten Dichtelement 10 bestehenden Verschluss 9 lösbar festhält. Auf der gegenüberliegenden Seite des Gehäuseteils 4 ist ein Transferset 16 eingesetzt, das sich von dem in Figur 3 dargestellten Transferset dadurch unterscheidet, dass eine Vertiefung 22, in der sich die das karpulenseitige Ende 20 der Verbindungskanüle 18 befindet, durch eine Membrane 15 gegen außen bakteriendicht verschlossen ist. Die aus medizinischem Sterilpapier bestehende Membrane 15 ist zu diesem Zweck mit Polyethylen beschichtet und mit dem Transferset 16 dicht verschweißt. Wie beim Beispiel nach Figur 3 ist das Transferset 16 durch einen O-Ring 53 im vorderen Gehäuseteil 4 abgedichtet und durch Bremsfahnen 54 in der dargestellten Position festgehalten. Beim Zusammenbau mit einer Aktivatoreinheit bleibt die Membrane 15 zunächst unversehrt. Erst bei der Aktivierung der Vorrichtung, genauer gesagt beim Vorschieben der Karpule 12, wie es im Zusammenhang mit den Figuren 5b und 5c beschrieben wurde, wird die Membrane 15 durch die Karpule 12 durchstoßen.

Eine weitere Ausführungsart der Vorrichtung ist in Figur 7 dargestellt. Wie bei den anhand der Figuren 1 bis 3 beschriebenen Ausführungsarten besteht auch hier das Gehäuse 3 aus dem vorderen Gehäuseteil 4 und dem die Karpule 12 enthaltenden hinteren Gehäuseteil 5, die an der Verbindungsstelle 8 miteinander verschraubt sind. Am hinteren Ende des hinteren Gehäuseteils 5 ist ein radial nach innen ragender Flansch 56 angeformt. Auf diesem ist eine Membrane 55 aus mit Polyethylen beschichtetem, medizinischem Sterilpapier dicht angeschweißt. Damit ist eine bakteriendichte Injektionseinheit gebildet. Rechts in der Figur 7 ist eine Betätigungseinheit dargestellt, die aus einem die Kolbenstange 29 tragenden Kupplungsteil 58 besteht. Letzterer weist eine Fingerauflage 32 auf und ist mittels einer Bajonettkupplung 59 mit dem hinteren Gehäuseteil 5 verbindbar. Wie bei den vorangehend beschriebenen Ausführungsarten ist die Kolbenstange 29 in einem Bremszylinderansatz 41 geführt. Am hinteren Ende des Bremszylinderansatzes 41 sind Rastmittel 60 angeordnet, die aus radial nach innen ragenden Rastnasen bestehen, die in an der Kolbenstange 29 vorhandene Kerben ragen und dafür sorgen, dass zum verschieben die Kolbenstange 29 ein gewisser Kraftaufwand erforderlich ist. Stirnseitig an der Kolbenstange 29 sind Schneidmittel 57 angeordnet, welche die Membrane 55 durchtrennen, sobald die Betätigungseinheit mittels der Bajonettverbindung 59 mit dem hinteren Gehäuseteil 5 verbunden wird.

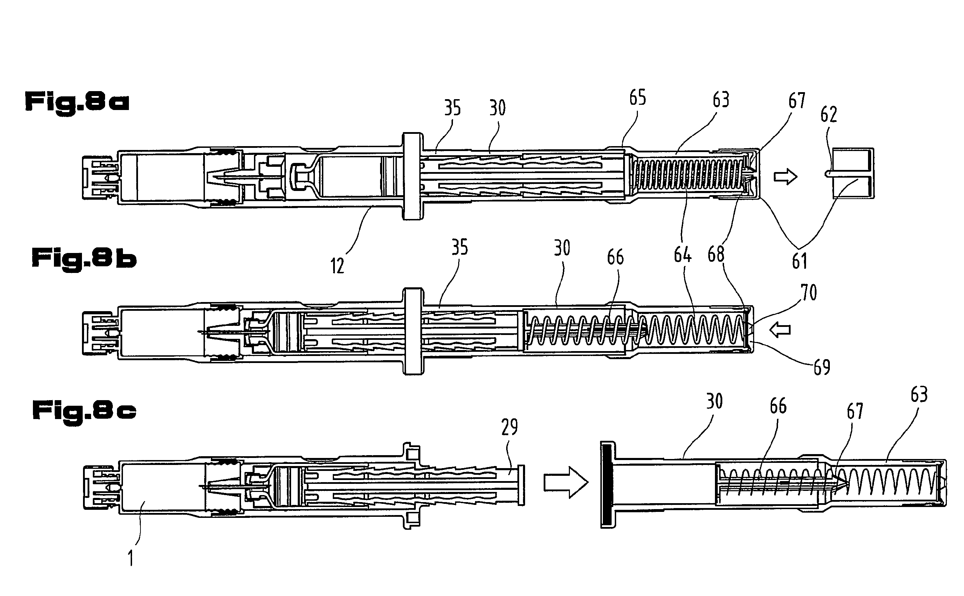

In den Figuren 8a bis 8c ist eine weitere Ausführungsart der Vorrichtung dargestellt, bei welcher die Aktivierung automatisch durch Federkraft erfolgt. Die Vorrichtung entspricht im Wesentlichen jener gemäß Figur 2, lediglich das hintere Ende des Kolbenstangengehäuses 30 ist anders gestaltet, wie dies im Folgenden beschrieben wird. Mit dem offenen, hinteren Ende des Kolbenstangengehäuses 30 ist mittels einer geschweißten, geschraubten oder geschnappten Verbindung 65 ein Federgehäuse 63 verbunden. Dieses trägt in seinem Inneren einen Stößel 66, der an seinem vorderen Ende tellerartig erweitert ist und an seinem hinteren Ende zwei Ratshaken 67 trägt. Im hinteren Ende des Federgehäuses 63 ist eine Rastscheibe 68 aufgenommen, die eine Durchgangsöffnung hat, in welcher die Rasthaken 67 in der in Figur 8a gezeigten Ausgangsposition sitzen. Eine Feder 64 sitzt auf dem Stößel 66 und ist zwischen dessen tellerartigen Erweiterung und der Rastscheibe 68 vorgespannt. Am hinteren Ende ist das Federgehäuse 63 mit einer Endkappe 69 abgeschlossen, die eine zentrale Öffnung 70 aufweist. Auf der Endkappe ist in der Ausgangsposition gemäß Figur 8a eine Sicherungskappe 61 aufgesetzt, die einen zentralen Sicherungsstift 62 aufweist, der durch die Öffnung 70 der Endkappe 69 hindurch bis zwischen die Rasthaken 67 reicht. Durch den Sicherungsstift 62 werden die Rasthaken 67 daran gehindert, sich gegeneinander zu bewegen und in der Folge durch die Öffnung in der Rastscheibe zu schlüpfen. dadurch ist die Vorrichtung in dieser Position gemäß Figur 8a gesichert und kann nicht ausgelöst werden. Am Übergang zwischen der Sicherungskappe 61, der Endkappe 69 und dem Federgehäuse 63 kann eine Etikette (nicht dargestellt) angebracht sein, die als Garantiesiegel dient. Es ist auch möglich, mit einer einzigen durchgehenden Etikette die Verbindungsstellen zwischen dem hinteren Gehäuseteil 5 und dem Kolbenstangengehäuse 30, zwischen dem Kolbenstangengehäuse 30 und dem Federgehäuse 63 sowie zwischen dem Federgehäuse 63, der Endkappe 69 und der Sicherungskappe 61 zu sichern.

Zur Vorbereitung für den Gebrauch wird zuerst die Sicherungskappe 61 abgezogen, wie dies durch den Pfeil in Figur 8a angedeutet ist. Nun kann die Aktivierung der Vorrichtung ausgelöst werden. Dies geschieht, indem die Endkappe in Richtung zum Abgabeende der Vorrichtung hin gedrückt wird. Dies hat zur Folge, dass die in der Endkappe 69 vorhandene, konisch ausgebildete Öffnung 70 die Rasthaken 76 gegeneinander drückt, so dass sie sich unter der Kraft der vorgespannten Feder 64 durch die Öffnung der Rastscheibe 68 hindurch bewegen. Ein Vergleich zwischen den Figuren 8a und 8b zeigt deutlich, dass die Endkappe 69 zwecks Auslösung der Vorrichtung einen axialen Weg zurücklegt. Die Figur 8b zeigt eine Position, die bezüglich der Injektionseinheit der Position gemäß Figur 4d entspricht. Das heißt, dass die Kolbenstange 29 sich unter der Kraft der Feder 64 so weit bewegt hat, dass das Lyophilisat in der ersten Kammer 1 mit dem aus der Karpule 12 in die erste Kammer 1 geflossene Lösungsmittel rekonstituiert wurde und - nach Entlüftung der ersten Kammer - zur Verabreichung bereit ist. Natürlich ist die Feder 64 so dimensioniert, dass ihre Kraft groß genug ist, um den Widerstand zu überwinden, der durch das Gaspolster in der ersten Kammer 1 und die gegenseitige Reibung der bewegten Bauteile der Vorrichtung entsteht. Wie bei den vorher beschriebenen Ausführungsarten verhindern auch hier an der Kolbenstange 29 vorgesehene Rasten 40, dass die Kolbenstange 29 zurück bewegt werden kann.

Nun kann das Kolbenstangengehäuse 30 mitsamt dem Federgehäuse und dem in diesem angeordneten Bauteilen von der Vorrichtung abgenommen werden, wie dies in Figur 8c dargestellt und durch einen Pfeil verdeutlicht ist.

Um auch bei dieser Ausführungsart der Vorrichtung die Sterilisierung mit Ethylenoxid zu ermöglichen, kann eine mit einer Membran versiegelte Öffnung (nicht dargestellt) im Mantel des Kolbenstangengehäuses 30, im Mantel des Federgehäuses 63 oder in der Sicherungskappe 61 vorgesehen sein.

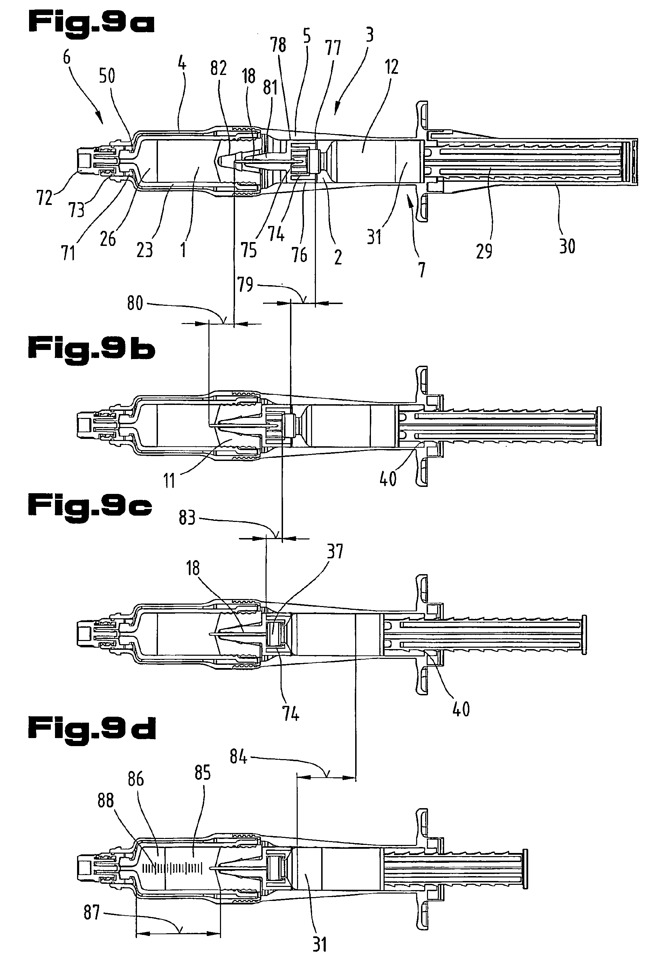

In den Figuren 9a bis 9e ist die Benutzung einer weiteren Ausführungsart der erfindungsgemäßen Vorrichtung beschrieben. Die Vorrichtung ist ähnlich aufgebaut wie die weiter oben unter Bezugnahme auf Fig. 1 beschriebene Vorrichtung, die wichtigsten Unterschiede werden nachstehend ebenfalls erläutert.

Die Vorrichtung weist ein aus einem vorderen Gehäuseteil 4 und einem hinteren Gehäuseteil 5 bestehendes Gehäuse auf, wobei im vorderen Gehäuseteil 4 ein Glaszylinder 23 untergebracht ist, welcher das Lyophilisat 26 enthält. An seinem injektionsseitigen Ende ist der Glaszylinder 23 mit einem Adapter 71 versehen, der in die Nut 50 des Glaszylinders 23 eingreift. Auf dem Adapter 71 sitzt ein Verschluss 72, der den Glaszylinder 23 dicht verschließt. Zwischen der Außenseite des Adapters 71 und der Innenseite des Halses des vorderen Gehäuseteils 4 ist ein O-Ring 73 angeordnet. Dieser Aufbau erlaubt es, den das Lyophilisat 26 enthaltenden und mit dem Adapter 71 und dem Verschluss 72 verschlossenen Glaszylinder 23 in den vorderen Gehäuseteil 4 einzusetzen. Beim Sterilisieren mit Ethylenoxyd kann dieses in Folge des Spiels zwischen dem vorderen Gehäuseteil 4 und dem Glaszylinder 23 bis zum O-Ring 73 vordringen und dieser hält den gesamten Innenraum dicht und steril.

Das Transferset 75 unterscheidet sich vom Transferset 16 gemäß Fig. 1 dadurch, dass es neben äußeren, mit der Innenwand des hinteren Gehäuseteils 5 zusammenwirkenden Federrastlappen 76 auch innere, mit dem Karpulenkopf zusammenwirkende Federrastlappen 74 hat. In der Ausgangsposition gemäß Fig. 9a sitzt die Karpule 12 mit dem Karpulenkopf auf inneren Federrastlappen 74 des Transfersets 75 auf. Die äußeren Federrastlappen 76 des Transfersets 75 sind in dieser Stellung in einer ersten, beispielsweise durch eine Ringnut 77 im hinteren Gehäuseteil 5 gebildeten Rastposition arretiert.

Die äußeren und inneren Federrastlappen sind dabei so dimensioniert, dass die Kraft zum Überwinden der Bremswirkung der äußeren Federrastlappen 76 in einem Bereich von beispielsweise 1 bis 1,5 Newton liegt und damit kleiner ist als die Kraft zum Überwinden der Bremswirkung der inneren Federrastlappen, welche beispielsweise etwa 2 Newton beträgt.

Ausgehend von der Position gemäß Fig. 9a wird zuerst das Kolbenstangengehäuse 30 entfernt, so dass die Kolbenstange frei liegt und betätigt werden kann. Zu Beginn der Betätigung drückt die Kolbenstange 29 auf den Karpulenstopfen 31 und schiebt die Karpule 12 mit dem Transferset 75 um den Weg 79 nach vorne. Dabei lösen sich zwangsläufig die äußeren Federrastlappen 76 des Transfersets 75 aus der Ringnut 77 und rasten im Zuge der Vorwärtsbewegung in der Ringnut 78 des hinteren Gehäuseteils 5 wieder ein.

Gleichzeitig wird der Zylinderstopfen 11 mit der Verbindungskanüle 18 durchstochen, welche den dem Weg 79 entsprechenden Weg 80 zurücklegt, sodass jetzt die vordere Kanülenspitze innerhalb der ersten Kammer 1 des Glaszylinders 23 zu stehen kommt. Dabei dichtet der Nadelansatzteil 81 des Transfersets 75 die konische Ausnehmung 82 des Zylinderstopfens 11 ab. Am Nadelansatzteil 81 sind Federelemente vorgesehen, die bei diesem Schritt in der konischen Ausnehmung 82 des Zylinderstopfens 11 einrasten und so das Transferset 75 mit dem Zylinderstopfen 11 verbinden. Dabei wird insbesondere verhindert, dass sich das Transferset 75 vom Zylinderstopfen 11 wieder entfernt und dabei Flüssigkeit in den Raum zwischen dem hinteren Gehäuseteil 5 und der Karpule 12 fließt. Die nun erreichte Position gemäß Fig. 9b wird durch Einrasten einer Raste 40 der Kolbenstange 29 gesichert.

Im weiteren Bewegungsablauf, von der in Fig. 9b dargestellten Position in die in Fig. 9c dargestellte Position, wird die Bremskraft der inneren Federrastlappen 74, welche kleiner ist als die Festsitzkraft des Zylinderstopfens 11 im Glaszylinder 23, überwunden und die Karpule 12 verschiebt sich relativ zum Transferset 75 um den Weg 83, bis sie mit dem Karpulenkopf dicht am Boden des Transfersets 75 aufsitzt. Dabei durchsticht die hintere Spitze der Verbindungskanüle 18 die im Karpulenkopf sitzende Karpulen-Dichtscheibe 37, so dass die Überleitung des Lösungsmittels durch die Verbindungskanüle 18 in die erste Kammer 1 jetzt eingeleitet werden kann. Die Verbindung zwischen der Verbindungskanüle 18 und der Karpule 12 in dieser Position wird durch eine weitere Raste 40 der Kolbenstange 29 abgesichert.

Im weiteren Bewegungsablauf von der Position gemäß Fig. 9c in die Position gemäβ Fig. 9d wird die Kolbenstange 29 langsam weiter gedrückt. Die Rasten 40 der Kolbenstange 29 und deren Führung im Bremszylinderansatz fungieren dabei als Bremse, die eine zu schnelle Vorwärtsbewegung der Kolbenstange 29 verhindert. Nach dem Zurücklegen des Weges 84 signalisiert ein spürbares Ansteigen des Widerstands, dass nun die Position gemäß Fig. 9d erreicht ist und das Lösungsmittel aus der Karpule 12 in die erste Kammer 1 der übergeführt wurde. In der ersten Kammer 1 wird das Lyophilisat 26 aufgelöst. Beim Einströmen des Lösungsmittels in die erste Kammer 1 baut sich in dieser ein Druck auf, der in Verbindung mit der beschriebenen Bremswirkung des Kolbenstangen-Rastensystems einem zu raschen Einspritzen des Lösungsmittels entgegenwirkt, so dass eine Schaumbildung während der Überleitung des Lösungsmittels in die erste Kammer 1 weitgehendst vermieden werden kann. Die Position des Zylinderstopfens 11 in der ersten Kammer 1, in Verbindung mit dem Transferset 75, wird dabei durch eine Kolbenstangenraste 40 abgesichert. Nach der Rekonstituierung des Wirkstoffes (Arzneimittel) wir der Injector sicherheitshalber leicht geschüttelt und die Lösung auf Partikel überprüft.

In der Position gemäß Fig. 9d enthält die erste Kammer 1 nun den rekonstituierten Wirkstoff 85 und Luft 86. Der Injector ist in diesem Moment so zu halten, dass der Verschluss 72 oben ist. Erst jetzt wird der Verschluss 72 vom Injector abgenommen und die Injektionskanüle 49 aufgesetzt. Die Sterilität des Injectors bzw. der Injektionslösung ist bis zum Aufsetzen der Injektionskanüle 49 sichergestellt. Nach dem Aufsetzten der Injektionskanüle 49 wird die Luft verdrängt und die erforderliche Lösungsmenge injiziert, wobei der Weg 87 zurückgelegt wird. Damit je nach Bedarf auch nur eine Teilmenge des Wirkstoffes 85 injiziert werden kann, ist auf dem Glaszylinder 23 oder auf dem vorderen Gehäuseteil 4 eine Skala 88 angeordnet. Nach dem Ausspritzen der Lösungsmenge rastet die entsprechende Sicherungsraste 40 der Kolbenstange 29 automatisch im Karpulengehäuse 3 ein und sichert die Kolbenstange 29 in der Endposition ab. Damit ist eine Manipulation (Herausziehen der Kolbenstange) mit dem Injector nicht mehr möglich.

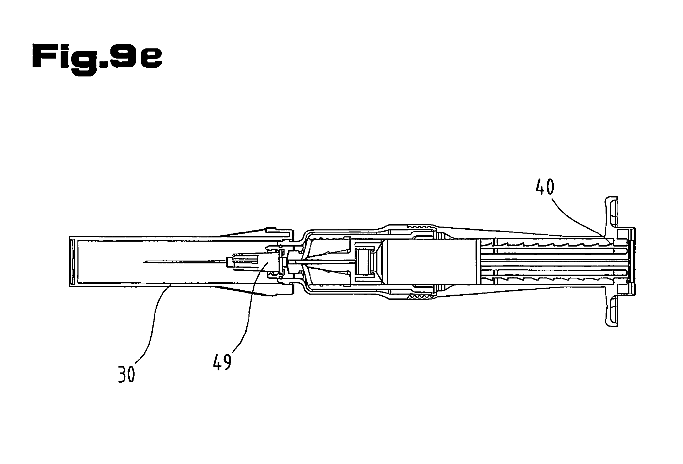

Um Verletzungen durch die kontaminierte Injektionsnadel 49 zu vermeiden, wird nach erfolgter Injektion das Kolbenstangengehäuse 30 einfach über die Kanüle 49 auf den vorderen Gehäuseteil 4 aufgesetzt, wie dies in Fig. 9e dargestellt ist, und verrastet dabei unlösbar. Der jetzt beidseitig verschlossene Injector, welcher nicht mehr manipulierbar ist, kann jetzt problemlos und ohne Verletzungsrisiko entsorgt werden.

Der Ordnung halber sei abschließend darauf hingewiesen, dass zum besseren Verständnis des Aufbaus der Vorrichtung diese bzw. deren Bestandteile teilweise unmaßstäblich und/oder vergrößert und/oder verkleinert dargestellt wurden.
Die den eigenständigen erfinderischen Lösungen zugrunde liegende Aufgabe kann der Beschreibung entnommen werden.

Vor allem können die einzelnen in den Figuren gezeigten Ausführungen den Gegenstand von eigenständigen, erfindungsgemäßen Lösungen bilden. Die diesbezüglichen, erfmdungsgemäßen Aufgaben und Lösungen sind den Detailbeschreibungen dieser Figuren zu entnehmen.

### Bezugszeichenaufstellung

- 1: erste Kammer
- 2: zweite Kammer
- 3: Gehäuse
- 4: vorderer Gehäuseteil
- 5: hinterer Gehäuseteil

- 6: vorderes Ende 3
- 7: hinteres Ende 3
- 8: Verbindungsstelle 4-5
- 9: Verschluss
- 10: Dichtelement in 9

- 11: Zylinderstopfen / Kolben
- 12: Karpule
- 13: Öffnung
- 14: Membrane in 13
- 15: weitere Membrane

- 16: Verbindungsmittel
- 17: Kanülenführung
- 18: Verbindungskanüle
- 19: Kanülenende Abgabeseite
- 20: Kanülenende Karpulenseite

- 21: Kanülenschutzkappe
- 22: Vertiefung
- 23: Glaszylinder
- 24: Mündung 23
- 25: Adapter

- 26: Lyophilisat
- 27: Verschlusskappe
- 28: Auflagegitter
- 29: Kolbenstange
- 30: Kolbenstangengehäuse

- 31: Karpulenstopfen
- 32: Fingerauflage
- 33: Verbindungsflansch
- 34: Etikette über 33
- 35: Etikette über 4+5

- 36: Karpulenschulter
- 37: Karpulen-Dichtscheibe
- 38: Bördelkappe auf 12
- 39: Karpulenauflage
- 40: Rasten

- 41: Bremszylinderansatz
- 42: Verschweißung bei 33
- 43: Bremswulst an 11
- 44: Bremssteg
- 45: Bypässe

- 46: Arretierungsansatz an 27
- 47: Federrastlappen
- 48: Ringnut
- 49: Injektionskanüle
- 50: Nut in 23

- 51: Kupplungsansatz
- 52: Verbindungskanülenaufnahme
- 53: O-Ring
- 54: Bremsfahnen
- 55: Membrane

- 56: Flansch
- 57: Schneidmittel
- 58: Kupplungsteil
- 59: Bajonettkupplung
- 60: Rastmittel

- 61: Sicherungskappe
- 62: Sicherungsstift
- 63: Federgehäuse
- 64: Feder
- 65: Verbindung

- 66: Stößel
- 67: Rasthaken
- 68: Rastscheibe
- 69: Endkappe
- 70: Öffnung

- 71: Adapter
- 72: Verschluss
- 73: O-Ring
- 74: innere Federrastlappen
- 75: Transferset

- 76: äußere Federrastlappen
- 77: Ringnut
- 78: Ringnut
- 79: Weg
- 80: Weg

- 81: Nadelansatzteil
- 82: konische Ausnehmung in 11
- 83: Weg
- 84: Weg
- 85: Wirkstoff

- 86: Luft
- 87: Weg
- 88: Skala

## Patentansprüche

1. Vorrichtung zum Lyophilisieren, Rekonstituieren und Verabreichen eines rekonstituierten Wirkstoffes, mit einem länglichen Gehäuse (3) mit einem abgabeseitigen vorderen Gehäuseende (6) und einem diesem gegenüberliegenden hinteren Gehäuseende (7), wobei in dem Gehäuse (3) im Bereich des vorderen Gehäuseendes (6) eine erste Kammer (1) mit einem darin enthaltenen Lyophilisat ausgebildet oder angeordnet ist, welche erste Kammer (1) an ihrem dem vorderen Gehäuseende (6) zugewandten Ende mit einem abnehmbaren Verschluss (9) und in Richtung des hinteren Gehäuseendes (7) mit einem Kolben (11) dicht verschlossen ist, wobei in dem Gehäuse (3) im Bereich des hinteren Gehäuseendes (7) eine zweite Kammer (2) ausgebildet oder angeordnet ist und wobei mindestens eine Öffnung (13) die zweite Kammer (2) mit der umgebenden Atmosphäre verbindet und durch eine Membrane (14; 55) verschlossen ist, die für Gas, jedoch nicht für Bakterien durchlässig ist, wobei in der zweiten Kammer (2) eine ein Lösungsmittel enthaltende Karpule (12) angeordnet ist und zwischen der ersten Kammer (1) und der Karpule (12) Verbindungsmittel (16; 75) angeordnet sind, die zum Herstellen einer Verbindung zwischen dem Inneren der Karpule (12) und der ersten Kammer (1) bestimmt sind, **dadurch gekennzeichnet, dass** die Karpule (12) an ihrer vom vorderen Gehäuseende (6) abgewandten Seite mit einem in der Karpule (12) verschiebbaren Karpulenstopfen (31) dicht verschlossen ist und dass am hinteren Gehäuseende (7) zum Zusammenwirken mit dem Karpulenstopfen (31) bestimmte Betätigungsmittel (29) angeordnet sind, die mit Rastmitteln (40, 41) ausgestattet sind, welche eine Bewegung der Betätigungsmittel (29) nur in Richtung zum vorderen Gehäuseende (6) gestatten und eine entgegengesetzte Bewegung verhindern.

2. Vorrichtung nach Anspruch 1, **dadurch gekennzeichnet, dass** der Kolben (11) auf der der ersten Kammer (1) zugewandten Seite der Membrane (55) dieser nahe benachbart angeordnet ist.

3. Vorrichtung nach Anspruch 2, **dadurch gekennzeichnet, dass** der Kolben (11) in einem Abstand von 0,1 bis 30 mm, bevorzugt 0,1 bis 5 mm, Abstand von der Membrane (55) angeordnet ist.

4. Vorrichtung nach einem der Ansprüche 1 bis 3, **dadurch gekennzeichnet, dass** das Volumen in der ersten Kammer (1) größer ist als das Volumen des lyophilisierten Wirkstoffes.

5. Vorrichtung nach einem der Ansprüche 1 bis 4, **dadurch gekennzeichnet, dass** in der ersten Kammer (1) der Luftdruck höher ist als 1 bar oder der Umgebungsluftdruck.

6. Vorrichtung nach einem der Ansprüche 1 bis 4, **dadurch gekennzeichnet, dass** der Luftdruck in der ersten Kammer (1) geringer ist als 1 bar oder der Umgebungsluftdruck.

7. Vorrichtung nach einem der Ansprüche 1 bis 6, **dadurch gekennzeichnet, dass** die Verbindungsmittel (16; 75) eine im Gehäuse (3) verschiebbare Kanfülenführung (17) umfassen, in der eine Verbindungskanüle (18) mit einem gegen den Kolben (11) gerichteten Kanülenende (19) und einem gegen die Karpule (12) gerichteten Kanülenende (20) aufgenommen ist und Verbindungskanüle (18) eine doppelendige Nadel ist, die in ihrem Mittelbereich in der Kanülenführung befestigbar ist und beispielsweise eingeklebt, eingeschäumt oder durch einen Presssitz gehaltert ist.

8. Vorrichtung nach einem der Ansprüche 1 bis 6, **dadurch gekennzeichnet, dass** die Verbindungsmittel (16; 75) eine Verbindungskanüle (18) umfassen, die an einem Kanülenende (19) im Kolben (11) aufgenommen ist, wobei das andere Kanülenende (20) gegen die Karpule (12) gerichtet ist.

9. Vorrichtung nach Anspruch 8, **dadurch gekennzeichnet, dass** das gegen die Karpule (12) gerichtete Kanülenende (20) mit einer Kappe (21) aus einem gummielastischen Material bedeckt ist, wobei die Kappe (21) mit dem Kolben (11) dicht verbunden ist.

10. Vorrichtung nach Anspruch 8, **dadurch gekennzeichnet, dass** das gegen die Karpule (12) gerichtete Kanülenende (20) in einer Vertiefung (22) des Kolbens (11) angeordnet ist und dass diese Vertiefung (22) durch eine weitere Membrane (15) verschlossen ist, die für Gas, jedoch nicht für Bakterien durchlässig ist.

11. Vorrichtung nach einem der vorangehenden Ansprüche, **dadurch gekennzeichnet, dass** die erste Kammer (1) von einem Glaszylinder (23) gebildet wird, der seinerseits im Gehäuse (3) aufgenommen ist.

12. Vorrichtung nach Anspruch 11, **dadurch gekennzeichnet, dass** der Glaszylinder (23) am abgabeseitigen Ende eine verengte Mündung (24) aufweist, die einen Adapter (25) trägt, auf den der Verschluss (9) aufgesetzt ist.

13. Vorrichtung nach einem der vorangehenden Ansprüche, **dadurch gekennzeichnet, dass** der Verschluss (9) ein Dichtelement (10) aus einem gummielastischen Material enthält, das bei aufgesetztem Verschluss (9) eine Mündungsöffnung der ersten Kammer (1) verschließt

14. Vorrichtung nach einem der vorangehenden Ansprüche, **dadurch gekennzeichnet, dass** der Verschluss (9) Durch eine Verrastung gegen unbeabsichtigtes Lösen gesichert ist

15. Vorrichtung nach einem der vorhergehenden Ansprüche, **dadurch gekennzeichnet, dass** der Verschluss (9) einen verschließbaren, für Luft, jedoch nicht für Flüssigkeiten passierbaren, mit dem Inneren der ersten Kammer (1) in Verbindung stehenden Auslass aufweist.

16. Vorrichtung nach Anspruch 15, **dadurch gekennzeichnet, dass** der Auslass bakteriendicht verschließbar ist.

17. Vorrichtung nach einem der Ansprüche 15 bis 16, **dadurch gekennzeichnet, dass** der Auslass über eine Originalitätssicherung mit dem Verschluss (9) bzw. dem Gehäuse (3) verbunden ist.

18. Vorrichtung nach einem der vorhergehenden Ansprüche, **dadurch gekennzeichnet, dass** der Verschluss (9) über eine Originalitätssicherung mit dem Gehäuse (3), der Mündungsöffnung oder dem Glaszylinder (23) verbunden ist.

19. Vorrichtung nach einem der vorangehenden Ansprüche, **dadurch gekennzeichnet, dass** das Gehäuse (3) aus zwei axial hintereinander angeordneten, miteinander an einer Verbindungsstelle (8) verbundenen Gehäuseteilen (4, 5) besteht, wobei sich die Verbindungsstelle (8) zwischen der ersten Kammer (1) und der zweiten Kammer (2) befindet.

20. Vorrichtung nach Anspruch 19, **dadurch gekennzeichnet, dass** die Verbindungsstelle (8) durch eine Gewindeverbindung gebildet ist.

21. Vorrichtung nach Anspruch 20, **dadurch gekennzeichnet, dass** die Verbindungsstelle (8) durch eine Originalitätssicherung, beispielsweise eine Etikette (35) gesichert ist.

22. Vorrichtung nach einem der vorangehenden Ansprüche, **dadurch gekennzeichnet, dass** das die erste Kammer (1) an ihrem dem vorderen Gehäuseende (6) abgewandten Ende im Durchmesser zum Innenraum Längsnuten (45) aufweist.

23. Vorrichtung nach einem der Ansprüche 1 bis 21, **dadurch gekennzeichnet, dass** der Kolben (11) aus einem gummielastischen Material besteht, einen größeren Durchmesser hat als der Innendurchmesser der ersten Kammer (1) und mindestens einen axial verlaufenden, im Bereich seines Umfangs angeordneten Entlüftungsdurchgang aufweist, dessen Querschnitt so dimensioniert ist, dass er durch die elastische Verformung des Kolbens (11) verschlossen ist, wenn dieser ganz in der ersten Kammer (1) aufgenommen ist.

24. Vorrichtung nach einem der Ansprüche 1 bis 9, **dadurch gekennzeichnet, dass** die durch die Membrane (14) verschlossene Öffnung (13) stirnseitig am hinteren Gehäuseende (7) angeordnet ist.

25. Vorrichtung nach Anspruch 24, **dadurch gekennzeichnet, dass** ein auf das hintere Gehäuseende (7) aufsetzbarer Kupplungsteil (58) vorgesehen ist, mit dem die Betätigungsmittel (29) verbunden sind, und dass am Kupplungsteil oder den Betätigungsmitteln Schneid- oder Trennmittel (57) angeordnet sind, die beim Aufsetzen des Kupplungsteils (58) auf das hintere Gehäuseende (7) die Membrane (14) durchdringen.

26. Vorrichtung nach einem der Ansprüche 1 bis 24, **dadurch gekennzeichnet, dass** die Betätigungsmittel (29) in einem Schutzrohr (30) aufgenommen sind, das lösbar und dicht am Gehäuse (3) befestigt ist.

27. Vorrichtung nach Anspruch 26, **dadurch gekennzeichnet, dass** die durch die Membrane (14) verschlossene Öffnung (13) im Schutzrohr angeordnet ist.

28. Vorrichtung nach einem der Ansprüche 1 bis 27, **dadurch gekennzeichnet, dass** die Verbindungsmittel (16; 75), der Kolbenstopfen (31) und der Kolben (11) so dimensioniert sind, dass während der Bewegung der Betätigungsmittel (29) in Richtung zum vorderen Gehäuseende (6) zuerst die Verbindungsmittel (16; 75) betätigt werden, so dass eine Strömungsverbindung von der Karpule (12) zur ersten Kammer (1) geschaffen wird, dann der Karpulenstopfen (31) in Richtung zum vorderen Gehäuseende (6) hin bewegt wird, so dass das Lösungsmittel aus der Karpule (12) in die erste Kammer (1) fließt und dort mit dem Lyophilisat gemischt wird und anschließend der Kolben (11) in der ersten Kammer (1) in Richtung zum vorderen Gehäuseende (6) hin bewegt wird, so dass der rekonstituierte Wirkstoff aus der ersten Kammer (1) ausgestoßen wird.

29. Vorrichtung nach Anspruch 28, **dadurch gekennzeichnet, dass** in Bewegungsrichtung der Betätigungsmittel (29) eine Mehrzahl unmittelbar aufeinander folgender angeordneter Rastmittel (40) angeordnet sind.

30. Vorrichtung nach Anspruch 29, **dadurch gekennzeichnet, dass** die Rastmittel durch eine Rastklinkenanordnung (40, 41) gebildet sind.

31. Vorrichtung nach Anspruch 30, **dadurch gekennzeichnet, dass** die Betätigungsmittel (29) eine Kolbenstange umfassen, die längs verlaufende, in radialer Richtung zur Längsmittelachse der Kolbenstange elastisch rückstellbar verformbare Stege aufweist und dass die Rastmittel Rasten (40) aufweisen, die auf der der Innenseite des Gehäuses zugewandten Stirnseite der Stege ausgebildet sind.

32. Vorrichtung nach einem der Ansprüche 28 bis 31, **dadurch gekennzeichnet, dass** die Verbindungsmittel (16; 75) mit einer, eine vorbestimmbare Rückhaltekraft aufweisenden ersten Rückhaltevorrichtung (47, 48; 76, 77, 78) im Gehäuse (3) im Innenraum des Gehäuses gehalten werden.

33. Vorrichtung nach Anspruch 32, **dadurch gekennzeichnet, dass** die erste Rückhaltevorrichtung durch eine in der Innenwand des Gehäuses vorgesehene, zumindest über einen Teil des Umfanges umlaufende Nut (48) und am Verbindungsmittel (16) angeordnete, in Richtung der Innenwand des Gehäuses vorgespannte Rastarme mit in die Nut eingreifenden Rastfortsätzen (47) gebildet ist.

34. Vorrichtung nach einem der vorangehenden Ansprüche, **dadurch gekennzeichnet**, der in der ersten Kammer (1) abdichtende Kolben (11) mit einer eine vorbestimmbare Rückhaltekraft aufweisenden Rückhaltevorrichtung versehen oder verbunden ist.

35. Vorrichtung nach Anspruch 34, **dadurch gekennzeichnet, dass** die Rückhaltevorrichtung durch einen am Kolben (11) angeordneten, einen größeren Durchmesser als den Innendurchmesser des Gehäuses aufweisenden Außendurchmesser aufweisenden Bremswalst (43) gebildet ist.

36. Vorrichtung nach Anspruch 35, **dadurch gekennzeichnet, dass** mehrere in Vorschubrichtung hintereinander angeordnete Bremswülste angeordnet sind.

37. Vorrichtung nach einem der Ansprüche 35 bis 36, **dadurch gekennzeichnet, dass** der Bremswulst (43) bzw. die Bremswülste in Vertiefungen in der Innenwand des Gehäuses eingreifen.

38. Vorrichtung nach einem der vorangehenden Ansprüche, **dadurch gekennzeichnet, dass** vorspannbare Federmittel (64) vorhanden sind, welche bei ihrer Entspannung die Bewegung der Betätigungsmittel (29) bewirken.

39. Vorrichtung nach Anspruch 32, **dadurch gekennzeichnet, dass** die erste Rückhaltevorrichtung (76, 77, 78) eine erste Rastposition (77) und eine zweite Rastposition (78) aufweist.

40. Vorrichtung nach Anspruch 39, **dadurch gekennzeichnet, dass** die Verbindungsmittel (75) eine zweite Rückhaltevorrichtung (74) aufweisen, die einer Relativbewegung zwischen der Karpule (12) und den Verbindungsmitteln (75) eine vorbestimmte Rückhaltekraft entgegen setzen.

41. Vorrichtung nach einem der Ansprüche 39 bis 40, **dadurch gekennzeichnet, dass** zwischen den Verbindungsmitteln (75) und dem Kolben (11) wirksame Rastmittel (81, 82) vorgesehen sind, die nach dem Herstellen der Strömungsverbindung zwischen der Karpule (12) und der ersten Kammer (1) eine Trennung der Verbindungsmittel (75) vom Kolben (11) verhindern.

42. Verfahren zum Bereitstellen eines Wirkstoffes in einer Vorrichtung nach einem der vorangehenden Ansprüche, **dadurch gekennzeichnet, dass** zuerst das der Abgabeseite gegenüberliegende Ende der ersten Kammer (1) mit dem Kolben (11) dicht verschlossen wird, dass der Wirkstoff durch die abgabeseitige Mündung (24) in die erste Kammer (1) eingefüllt und in der Kammer lyophilisiert wird und dass dann die Mündung durch den Verschluss (9) dicht verschlossen wird.

## Claims

1. Device for lyophilising, reconstituting and administering a reconstituted substance, with an elongate housing (3) comprising a dispensing-side front housing end (6) and a rear housing end (7) lying opposite it, and a first chamber (1) with a lyophilisate contained in it is provided or formed in the housing (3) in the region of the front housing end (6), which first chamber (1) is tightly sealed at its end facing the front housing end (6) by means of a removable closure (9) and by means of a plunger (11) in the direction of the rear housing end (7), and a second chamber (2) is provided or formed in the housing (3) in the region of the rear housing end (7), and at least one opening (13) connects the second chamber (2) to the ambient atmosphere and is closed by a membrane (14; 55) which is permeable to gas but not to bacteria, and a cartridge (12) containing a diluent is disposed in the second chamber (2), and connection means (16; 75) are disposed between the first chamber (1) and the cartridge (12) which are designed to establish a connection between the interior of the cartridge (12) and the first chamber (1), **characterised in that** the cartridge (12) is tightly sealed at its end remote from the front housing end (6) by means of a cartridge stopper (31) displaceable in the cartridge (12), and operating means (29) designed to co-operate with the cartridge stopper (31) are disposed on the rear housing end (7) and are provided with catch means (40, 41) which permit a movement of the operating means (29) in the direction towards the front housing end (6) only and prevent a movement in the opposite direction.

2. Device as claimed in claim 1, **characterised in that** the plunger (11) is disposed on the side of the membrane (55) facing the first chamber (1) and close to it.

3. Device as claimed in claim 2, **characterised in that** the plunger (11) is disposed at a distance of 0.1 to 30 mm, preferably 0.1 to 5 mm, from the membrane (55).

4. Device as claimed in one of claims 1 to 3, **characterised in that** the volume of the first chamber (1) is greater than the volume of the lyophilised substance.

5. Device as claimed in one of claims 1 to 4, **characterised in that** the air pressure in the first chamber (1) is higher than 1 bar or ambient air pressure.

6. Device as claimed in one of claims 1 to 4, **characterised in that** the air pressure in the first chamber (1) is lower than 1 bar or ambient air pressure.

7. Device as claimed in one of claims 1 to 6, **characterised in that** the connection means (16; 75) comprise a cannula guide (17) displaceable in the housing (3), in which a connecting cannula (18) with one cannula end (19) directed towards the plunger (11) and one cannula end (20) directed towards the cartridge (12) is accommodated, and the connecting cannula (18) is a double-ended needle which can be secured by its middle region in the cannula guide and is retained by gluing, foaming or by a press-fit seating, for example.

8. Device as claimed in one of claims 1 to 6, **characterised in that** the connection means (16; 75) comprise a connecting cannula (18) which is accommodated in the plunger (11) at one cannula end (19), and the other cannula end (20) is directed towards the cartridge (12).

9. Device as claimed in claim 8, **characterised in that** the cannula end (20) directed towards the cartridge (12) is covered by a cap (21) made from a rubber elastic material and the cap (21) is connected to the plunger (11) in a sealed arrangement.

10. Device as claimed in claim 8, **characterised in that** the cannula end (20) directed towards the cartridge (12) is disposed in a recess (22) of the plunger (11) and this recess (22) is closed by another membrane (15) which is permeable to gas but not to bacteria.

11. Device as claimed in one of the preceding claims, **characterised in that** the first chamber (1) is formed by a glass cylinder (23) which is in turn accommodated in the housing (3).

12. Device as claimed in claim 11, **characterised in that** the glass cylinder (23) has a tapered opening (24) at the dispensing-side end, which supports an adapter (25) fitted on the closure (9).

13. Device as claimed in one of the preceding claims, **characterised in that** the closure (9) contains a seal element (10) made from a rubber elastic material, which closes off an opening orifice of the first chamber (1) when the closure (9) is fitted. (9)

14. Device as claimed in one of the preceding claims, **characterised in that** the closure (9) is secured by a catch connection to prevent it from being inadvertently released.

15. Device as claimed in one of the preceding claims, **characterised in that** the closure (9) has an outlet communicating with the interior of the first chamber (1) which can be closed, through which air can pass but not liquids.

16. Device as claimed in claim 15, **characterised in that** the outlet can be closed to prevent ingress by bacteria.

17. Device as claimed in one of claims 15 to 16, **characterised in that** the outlet is connected to the closure (9) or to the housing (3) by an anti-tamper device.

18. Device as claimed in one of the preceding claims, **characterised in that** the closure (9) is connected to the housing (3), opening orifice or glass cylinder (23) by an anti-tamper device.

19. Device as claimed in one of the preceding claims, **characterised in that** the housing (3) comprises two housing parts (4, 5) disposed axially one behind the other and connected to one another at a connection point (8), and the connection point (8) is disposed between the first chamber (1) and the second chamber (2).

20. Device as claimed in claim 19, **characterised in that** the connection point (8) is provided in the form of a thread connection.

21. Device as claimed in claim 20, **characterised in that** the connection point (8) is secured by an anti-tamper device, for example a label (35).

22. Device as claimed in one the preceding claims, **characterised in that** the first chamber (1) has longitudinal grooves (45) in the diameter towards the interior at its end remote from the front housing end (6).

23. Device as claimed in one of claims 1 to 21, **characterised in that** the plunger (11) is made from a rubber elastic material and has a bigger diameter than the internal diameter of the first chamber (1) and has at least one axially extending venting passage disposed in the region of its circumference, the cross-section of which is dimensioned so that it is closed by the elastic deformation of the plunger (11) when the latter is fully accommodated in the first chamber (1).

24. Device as claimed in one of claims 1 to 21, **characterised in that** the opening (13) closed by the membrane (14) is disposed at the end of the rear housing end (7).

25. Device as claimed in claim 24, **characterised in that** a coupling part (58) is provided which can be fitted on the rear housing end (7), by means of which operating means (29) are connected, and cutting or severing means (57) are provided on the coupling part or on the operating means which pierce the membrane (14) when the coupling part (58) is fitted on the rear housing end (7).

26. Device as claimed in one of claims 1 to 24, **characterised in that** the operating means (29) are accommodated in a protective tube (30) which is releasably and sealingly attached to the housing (3).

27. Device as claimed in 26, **characterised in that** the opening (13) closed by the membrane (14) is disposed in the protective tube.

28. Device as claimed in one of claims 1 to 27, **characterised in that** the connection means (16; 75) of the plunger stopper (31) and the plunger (11) are dimensioned so that whilst the operating means (29) is being moved in the direction towards the front housing end (6), the connection means (16; 75) is operated first of all so that a flow connection is established from the cartridge (12) to the first chamber (1), the cartridge stopper (31) is then moved in the direction towards the front housing end (6) so that the diluent flows from the cartridge (12) into the first chamber (1) where it is mixed with the lyophilisate, and the plunger (11) in the first chamber (1) is then moved in the direction towards the front housing end (6) so that the reconstituted substance is ejected from the first chamber (1).

29. Device as claimed in claim 28, **characterised in that** a plurality of catch means (40) are disposed one immediately after the other in the direction of movement of the operating means (29).

30. Device as claimed in claim 29, **characterised in that** the catch means are provided in the form of a catch pawl arrangement (40, 41).

31. Device as claimed in claim 30, **characterised in that** the operating means (29) comprise a plunger rod with deformable webs extending along it which can elastically rebound in the direction extending radially with respect to the longitudinal mid-axis of the plunger rod, and the catch means have catches (40) formed on the terminal end of the webs facing the internal face of the housing.

32. Device as claimed in one of claims 28 to 31, **characterised in that** the connection means (16;75 ) are retained in the interior of the housing by means of a first retaining mechanism (47, 48; 76, 77, 78) in the housing (3) with a pre-definable retaining force.

33. Device as claimed in claim 32, **characterised in that** the first retaining mechanism is provided in the form of a groove (48) in the internal wall of the housing extending around at least a part of the circumference and catch arms disposed on the connection means (16) biased in the direction of the internal wall of the housing with catch projections (47) locating in the groove.

34. Device as claimed in one of the preceding claims, **characterised in that** the plunger (11) is provided with or connected to a retaining mechanism with a pre-definable retaining force in an arrangement sealing off the first chamber (1).

35. Device as claimed in claim 34, **characterised in that** the retaining mechanism is provided in the form of a brake bead (43) disposed on the plunger (11) which has an external diameter of a bigger diameter than the internal diameter of the housing.

36. Device as claimed in claim 35, **characterised in that** several brake beads are disposed one after the other in the direction of forward movement.

37. Device as claimed in one of claims 35 to 36, **characterised in that** the brake bead (43) or the brake beads locate in recesses in the internal wall of the housing.

38. Device as claimed in one of the preceding claims, **characterised in that** biased spring means (64) are provided which cause the movement of the operating means (29) when they are relaxed.

39. Device as claimed in claim 32, **characterised in that** the first retaining mechanism (76, 77, 78) has a first catch position (77) and a second catch position (78).

40. Device as claimed in claim 39, **characterised in that** the connection means (75) have a second retaining mechanism (74) which opposes a relative movement between the cartridge (12) and the connection means (75) by applying a pre-defined retaining force.

41. Device as claimed in one of claims 39 to 40, **characterised in that** active catch means (81, 82) are provided between the connection means (75) and the plunger (11), which prevent the connection means (75) from being separated from the plunger (11) once the flow connection has been established between the cartridge (12) and the first chamber (1).

42. Method of containing a substance in a device as claimed in one of the preceding claims, **characterised in that** the end of the first chamber (1) lying opposite the dispensing end is tightly sealed by the plunger (11) first of all, this first chamber (1) is filled with the substance through the dispensing-end opening (24) which is lyophilised in the chamber, and the opening is then tightly sealed by means of the closure (9).

## Revendications

1. Dispositif de lyophilisation, de reconstitution et d'administration d'un principe actif reconstitué, comportant un boîtier (3) allongé avec une extrémité avant (6) du côté administration et une extrémité arrière (7) opposée à celle-ci, une première chambre (1), contenant un lyophilisat, étant réalisée ou agencée dans le boîtier (3) dans la zone de l'extrémité avant (6), ladite première chambre (1) étant obturée hermétiquement avec une fermeture (9) amovible au niveau de son extrémité orientée vers l'extrémité avant (6) et avec un piston (11) en direction de l'extrémité arrière (7) du boîtier, une deuxième chambre (2) étant réalisée ou agencée dans le boîtier (3) dans la zone de son extrémité arrière (7) et ladite deuxième chambre (2) étant reliée à l'atmosphère ambiante par l'intermédiaire d'au moins une ouverture (13) et étant fermée par une membrane (14; 55) qui est perméable aux gaz, mais ne laisse pas passer les bactéries, une carpule (12), contenant un solvant, étant agencée dans la deuxième chambre (2), et des moyens de liaison (16; 75) étant disposés entre la première chambre (1) et la carpule (12) et étant destinés à établir une liaison entre l'intérieur de la carpule (12) et la première chambre (1), **caractérisé en ce que** la carpule (12), sur son côté opposé à l'extrémité avant (6) du boîtier, est obturée hermétiquement par un bouchon (31) mobile en translation dans la carpule (12), et **en ce qu'**au niveau de l'extrémité arrière (7) du boîtier sont disposés des moyens de manoeuvre (29) destinés à coopérer avec le bouchon (31) de la carpule et munis de moyens de blocage (40, 41) qui autorisent un mouvement des moyens de manoeuvre (29) uniquement vers l'extrémité avant (6) du boîtier et empêchent tout mouvement dans le sens opposé.

2. Dispositif selon la revendication 1, **caractérisé en ce que** le piston (11) est disposé à proximité de la membrane (55), sur le côté de celle-ci orienté vers la première chambre (1).

3. Dispositif selon la revendication 2, **caractérisé en ce que** le piston (11) est disposé à distance de la membrane (55), à une distance de 0,1 à 30 mm, de préférence de 0,1 à 5 mm.

4. Dispositif selon l'une quelconque des revendications 1 à 3, **caractérisé en ce que** le volume dans la première chambre (1) est supérieur au volume du principe actif lyophilisé.

5. Dispositif selon l'une quelconque des revendications 1 à 4, **caractérisé en ce que** la pression d'air dans la première chambre (1) est supérieure à 1 bar ou supérieure à la pression atmosphérique.

6. Dispositif selon l'une quelconque des revendications 1 à 4, **caractérisé en ce que** la pression d'air dans la première chambre (1) est inférieure à 1 bar ou inférieure à la pression atmosphérique.

7. Dispositif selon l'une quelconque des revendications 1 à 6, **caractérisé en ce que** les moyens de liaison (16; 75) comprennent un guidage de canule (17), qui est mobile en translation dans le boîtier (3) et dans lequel est logée une canule de jonction (18) avec une extrémité (19) orientée vers le piston (11) et une extrémité (20) orientée vers la carpule (12), et la canule de jonction (18) est une aiguille à double extrémité qui, dans sa partie centrale peut être fixée dans le guidage de canule et peut être maintenue, par exemple, par collage, par expansion ou par un ajustement serré.

8. Dispositif selon l'une quelconque des revendications 1 à 6, **caractérisé en ce que** les moyens de liaison (16; 75) comprennent une canule de jonction (18) logée avec son extrémité (19) dans le piston (11), l'autre extrémité (20) de la canule étant dirigée vers la carpule (12).

9. Dispositif selon la revendication 8, **caractérisé en ce que** l'extrémité (20) de la canule, dirigée vers la carpule (12), est fermée par un capuchon (21) en matériau élastique, ledit capuchon (21) étant relié de manière étanche au piston (11).

10. Dispositif selon la revendication 8, **caractérisé en ce que** l'extrémité (20) de la canule, dirigée vers la carpule (12), est disposée dans un creux (22) du piston (11), et **en ce que** ledit creux (22) est fermé par une autre membrane (15) qui est perméable aux gaz, mais ne laisse pas passer les bactéries.

11. Dispositif selon l'une quelconque des revendications précédentes, **caractérisé en ce que** la première chambre (1) est formée par un cylindre en verre (23) qui, pour sa part, est logé dans le boîtier (3).

12. Dispositif selon la revendication 11, **caractérisé en ce que** le cylindre en verre (23) comporte, au niveau de son extrémité d'administration, une bouche (24) rétrécie portant un adaptateur (25), sur lequel est posée la fermeture (9).

13. Dispositif selon l'une quelconque des revendications précédentes, **caractérisé en ce que** la fermeture (9) contient un élément d'étanchéité (10) en matériau élastique, qui obture un orifice d'embouchure de la première chambre (1) lorsque la fermeture (9) est en place.

14. Dispositif selon l'une quelconque des revendications précédentes, **caractérisé en ce que** la fermeture (9) est immobilisée par un système de blocage empêchant toute désolidarisation accidentelle.

15. Dispositif selon l'une quelconque des revendications précédentes, **caractérisé en ce que** la fermeture (9) comporte une sortie pouvant être fermée, laissant passer l'air, mais pas les liquides, communiquant avec l'intérieur de la première chambre (1).

16. Dispositif selon la revendication 15, **caractérisé en ce que** la sortie peut être obturée de manière étanche aux bactéries.

17. Dispositif selon l'une quelconque des revendications 15 à 16, **caractérisé en ce que** la sortie est reliée à la fermeture (9) ou au boîtier (3) par un témoin d'inviolabilité.

18. Dispositif selon l'une quelconque des revendications précédentes, **caractérisé en ce que** la fermeture (9) est reliée au boîtier (3), à l'orifice d'embouchure ou au cylindre de verre (23) par un témoin d'inviolabilité.

19. Dispositif selon l'une quelconque des revendications précédentes, **caractérisé en ce que** le boîtier (3) est formé par deux parties (4, 5) disposées axialement l'une derrière l'autre et reliées l'une à l'autre au niveau d'une zone d'assemblage (8), ladite zone d'assemblage (8) étant située entre la première chambre (1) et la deuxième chambre (2).

20. Dispositif selon la revendication 19, **caractérisé en ce que** la zone d'assemblage (8) est formée par un assemblage fileté.

21. Dispositif selon la revendication 20, **caractérisé en ce que** la zone d'assemblage (8) est sécurisée par un témoin d'inviolabilité, tel qu'une étiquette (35).

22. Dispositif selon l'une quelconque des revendications précédentes, **caractérisé en ce que** la première chambre (1), au niveau de son extrémité opposée à l'extrémité avant (6) du boîtier, comporte des rainures longitudinales (45) dans le diamètre vers le volume intérieur.

23. Dispositif selon l'une quelconque des revendications 1 à 21, **caractérisé en ce que** le piston (11) est réalisé en matériau élastique, possède un diamètre supérieur au diamètre intérieur de la première chambre (1) et comporte au moins un passage de purge d'air orienté axialement, disposé dans la zone de son pourtour et ayant une section transversale dimensionnée de telle sorte qu'il est obturé par la déformation élastique du piston (11) lorsque celui-ci est logé complètement dans la première chambre (1).

24. Dispositif selon l'une quelconque des revendications 1 à 9, **caractérisé en ce que** l'ouverture (13), obturée par la membrane (14), est disposée du côté frontal au niveau de l'extrémité arrière (7) du boîtier.

25. Dispositif selon la revendication 24, **caractérisé en ce qu'**il est prévu un élément de couplage (58), pouvant être posé sur l'extrémité arrière (7) du boîtier et auquel sont reliés les moyens de manoeuvre (29), et **en ce que** sur l'élément de couplage ou sur les moyens de manoeuvre sont disposés des moyens de coupe ou de séparation (57) qui transpercent la membrane (14) lorsque l'élément de couplage (58) est appliqué sur l'extrémité arrière (7) du boîtier.

26. Dispositif selon l'une quelconque des revendications 1 à 24, **caractérisé en ce que** les moyens de manoeuvre (29) sont logés dans un tube de protection (30) fixé de manière amovible et étanche sur le boîtier (3).

27. Dispositif selon la revendication 26, **caractérisé en ce que** l'ouverture (13), obturée par la membrane (14), est disposée dans le tube de protection.

28. Dispositif selon l'une quelconque des revendications 1 à 27, **caractérisé en ce que** les moyens de liaison (16; 75), le bouchon (31) du piston et le piston (11) sont dimensionnés de telle sorte que pendant le mouvement des moyens de manoeuvre (29) vers l'extrémité avant (6) du boîtier, sont actionnés d'abord les moyens de liaison (16; 75), de telle sorte qu'une liaison fluidique est établie depuis la carpule (12) vers la première chambre (1), ensuite le bouchon (31) de la carpule est déplacé vers l'extrémité avant (6) du boîtier, de telle sorte que le solvant coule hors de la carpule (12) vers l'intérieur de la première chambre (1) et s'y mélange au lyophilisat, ensuite, le piston (11) dans la première chambre (1) est déplacé vers l'extrémité avant (6) du boîtier de telle sorte que le principe actif reconstitué est expulsé hors de la première chambre (1).

29. Dispositif selon la revendication 28, **caractérisé en ce qu'**une pluralité de moyens de blocage (40) directement successifs sont disposés dans le sens de déplacement des moyens de manoeuvre (29).

30. Dispositif selon la revendication 29, **caractérisé en ce que** les moyens de blocage sont formés par un système de cliquets de blocage (40, 41).

31. Dispositif selon la revendication 30, **caractérisé en ce que** les moyens de manoeuvre (29) comprennent une tige de piston comportant des nervures déformables, orientées longitudinalement, pouvant être rappelées élastiquement dans la direction radiale par rapport à l'axe médian longitudinal de la tige de piston, et **en ce que** les moyens de blocage comportent des crans d'arrêt (40) qui sont réalisés sur la face frontale des nervures, orientée vers la face intérieure du boîtier.

32. Dispositif selon l'une quelconque des revendications 28 à 31, **caractérisé en ce que** les moyens de liaison (16; 75) sont maintenus dans le volume intérieur du boîtier par un premier dispositif de retenue (47, 48; 76, 77, 78), agencé dans le boîtier (3) et ayant une force de retenue prédéfinissable.

33. Dispositif selon la revendication 32, **caractérisé en ce que** le premier dispositif de retenue est formé par une rainure (48) prévue dans la paroi intérieure du boîtier sur au moins une partie du pourtour et par des bras de blocage, disposés sur le moyen de liaison (16), précontraints vers la paroi intérieure du boîtier et munis d'ergots de blocage (47) s'engageant dans la rainure.

34. Dispositif selon l'une quelconque des revendications précédentes, **caractérisé en ce que** le piston (11), obturant hermétiquement la première chambre (1), est muni ou relié à un dispositif de retenue ayant une force de retenue prédéfinissable.

35. Dispositif selon la revendication 34, **caractérisé en ce que** le dispositif de retenue est formé par un bourrelet de retenue (43), disposé sur le piston (11) et ayant un diamètre extérieur supérieur au diamètre intérieur du boîtier.

36. Dispositif selon la revendication 35, **caractérisé en ce que** plusieurs bourrelets de retenue sont disposés les uns derrière les autres dans le sens d'avance.

37. Dispositif selon l'une quelconque des revendications 35 à 36, **caractérisé en ce que** le bourrelet de retenue (43) ou les bourrelets de retenue s'engagent dans des creux ménagés dans la paroi intérieure du boîtier.

38. Dispositif selon l'une quelconque des revendications précédentes, **caractérisé en ce qu'**il est prévu des moyens de ressort (64) pouvant être précontraints et induisant, lors de leur détente, le mouvement des moyens de manoeuvre (29).

39. Dispositif selon la revendication 32, **caractérisé en ce que** le premier dispositif de retenue (76, 77, 78) comporte une première position de blocage (77) et une deuxième position de blocage (78).

40. Dispositif selon la revendication 39, **caractérisé en ce que** les moyens de liaison (75) comportent un deuxième dispositif de retenue (74) qui oppose une force de retenue prédéterminée au mouvement relatif entre la carpule (12) et les moyens de liaison (75).

41. Dispositif selon l'une quelconque des revendications 39 à 40, **caractérisé en ce qu'**entre les moyens de liaison (75) et le piston (11) sont prévus des moyens de blocage (81, 82) efficaces qui, après l'établissement de la liaison fluidique entre la carpule (12) et la première chambre (1), empêchent une séparation entre les moyens de liaison (75) et le piston (11).

42. Procédé de mise à disposition d'un principe actif dans un dispositif selon l'une quelconque des revendications précédentes, **caractérisé en ce qu'**en premier lieu, l'extrémité, opposée au côté d'administration, de la première chambre (1) est obturée hermétiquement avec le piston (11), **en ce que** le principe actif est introduit dans la première chambre (1) via la bouche (24) du côté admission et est lyophilisé dans la chambre, et **en ce que** la bouche est ensuite obturée hermétiquement par la fermeture (9).
